(19) Europäisches Patentamt
European Patent Office
Office européen des brevets

(11) EP 4 692 301 A1

(12) **EUROPEAN PATENT APPLICATION**
published in accordance with Art. 153(4) EPC

(43) Date of publication:
11.02.2026 Bulletin 2026/07

(21) Application number: 24777930.9

(22) Date of filing: 22.03.2024

(51) International Patent Classification (IPC):
$C12M\ 1/34^{(2006.01)}$  $C12M\ 1/00^{(2006.01)}$
$C12Q\ 1/02^{(2006.01)}$  $G06V\ 20/69^{(2022.01)}$

(52) Cooperative Patent Classification (CPC):
C12M 1/00; C12M 1/34; C12M 1/42; C12Q 1/02;
G01N 11/00; G01N 21/55; G01N 33/543;
G06V 20/69

(86) International application number:
PCT/CN2024/083362

(87) International publication number:
WO 2024/199141 (03.10.2024 Gazette 2024/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 24.03.2023  CN 202310299296
24.03.2023  CN 202310299292
24.03.2023  CN 202310299290
24.03.2023  CN 202310299306
24.03.2023  CN 202310299309
24.03.2023  CN 202310297791
24.03.2023  CN 202310299308

(71) Applicant: Yigong Ruixin (Xiamen) Technology
Co., Ltd
Xiamen, Fujian 361015 (CN)

(72) Inventor: LIN, Zhe
Xiamen, Fujian 361015 (CN)

(74) Representative: Chung, Hoi Kan
Mandarin IP Limited
7 Cherry Trees
Great Shelford
Cambridge CB22 5XA (GB)

(54) **CELL CHARACTERIZATION, TYPING AND IDENTIFICATION METHOD AND USE**

(57) The present invention relates to the field of biotechnology, and in particular to a method for characterizing, typing and identifying cells and use thereof. Cellular physical information includes cellular mechanical force and/or hardness obtained under at least one of the following conditions: an interaction between cells and/or multicellular aggregates, cells and/or multicellular aggregates at different growth times, different regions within multicellular aggregates, an effect of a substance on cells and/or multicellular aggregates, and an effect of other physical, biological or chemical factors on cells and/or multicellular aggregates. According to the present invention, typing and identification are performed on the cells and/or the multicellular aggregates through the above characterization method. The present invention characterizes real-time and continuous states of the cells and/or the multicellular aggregates through the cellular physical information, which can identify various types and states of cells and/or multicellular aggregates in a short time, at low cost and with high throughput, with an accuracy rate of over 98%; The present invention is further defined to be implemented through a characterization system, which can achieve the above effects.

FIG. 7

## Description

## Technical Field

The present invention relates to the field of biotechnology, and in particular to a method for characterizing, typing and identifying cells and use thereof.

## Background Art

In the biological field, for cells or multicellular aggregates in the drug development process, the selection of cell models is crucial because effective cell models can more accurately predict drug responses in vivo. Although traditional two-dimensional (2D) cell culture models are easy to operate and observe, their prediction results often have large gaps with the actual situation in vivo due to the lack of simulation of the in vivo microenvironment. In order to better simulate the in vivo microenvironment, tumor spheroids and organoids have received widespread attention as multicellular aggregate culture models in recent years. Especially, the characterization of cell-cell interactions is particularly important.

Cells are the basic unit of life. Their adhesion, migration, differentiation, apoptosis processes, dynamic changes in different physiological and pathological processes, and interactions with macromolecules are of great significance for understanding and regulating life phenomena. Therefore, characterizing the type, state, behavior, etc. of cells and multicellular aggregates is an important subject in the fields of bioengineering, cell biology, physical biology, etc.

At present, the main methods for characterizing cells and multicellular aggregates include: physical methods: using instruments to measure the mechanical or electrical properties of cells, such as mechanical force, adhesion force, elastic modulus, impedance, etc. These methods can reflect cell morphology, function and metabolic state, but existing technologies usually require expensive equipment and data processing techniques. For example, atomic force microscopy, mechanical force microscopy, impedance spectroscopy, etc. The operation is complex, the throughput is low, and the cost is high. Also, due to limitations such as throughput or phototoxicity, it is difficult to monitor cells for a long time and in real-time. Biochemical methods: using reagents or markers to measure cells. These methods can sensitively detect cell states and binding with macromolecules, but may also affect their own characteristics or functions. For example, fluorescence resonance energy transfer, biotin-avidin system, enzyme-linked immunosorbent assay, etc. Although chemical methods can sensitively detect the binding between cells and macromolecules, they usually require labeling or modification of targets, and may interfere with their normal functions.

## Summary of the Invention

(1) Technical Problem to be Solved

In view of the above shortcomings and deficiencies of the prior art, the present invention provides a method for characterizing cells, which can characterize cells and multicellular aggregates by detecting and acquiring static or dynamic cellular mechanical force and/or hardness of cells/multicellular aggregates at different times or before, during and after the action of other factors;

Correspondingly, the present invention also provides a method for cell typing and identification, which can realize typing and identification of cells and/or multicellular aggregates under any circumstances through cellular mechanical force and/or hardness.

Correspondingly, the present invention also provides use of the above method.

(2) Technical Solutions

In order to achieve the above objective, the main technical solutions adopted by the present invention include: In a first aspect, the present invention provides a method for characterizing cells, including characterizing cells and/or multicellular aggregates by acquiring cellular physical information of cells and/or multicellular aggregates.

Optionally, the cellular physical information includes cellular mechanical force and/or hardness acquired under at least one of the following conditions:

(1) an interaction between the cells and the multicellular aggregates;

(2) an interaction between the cells;

(3) an interaction between the multicellular aggregates;

(4) cells and/or multicellular aggregates at different growth times;

(5) different regions within the multicellular aggregates;

(6) an effect of a substance on the cells and/or the multicellular aggregates; and

(7) an effect of other physical, biological or chemical factors on the cells and/or the multicellular aggregates;

where the multicellular aggregate is a cell population formed by aggregation of more than two cells.

In a second aspect, the present invention also provides a method for typing and identifying cells and/or multicellular aggregates, including typing and identifying the cells and/or multicellular aggregates through the characterization method described above.

Wherein, typing of cells and/or multicellular aggregates refers to classification based on differences in characteristics including but not limited to type, state, behavior, spatial omics characteristics, etc. of cells and/or multicellular aggregates under different factors.

In a third aspect, the present invention also provides use of the method described in any scheme, including at least one of the following:

use in establishing an in vitro organ and a cell model;

use in screening an organ therapeutic drug, and in research on an organ model in physiological and pathological states;

use in a method for evaluating drug efficacy against a tumor and a related evaluation product; and

use in cell therapy, synthetic biology, adipose research, research on an interaction between cells/multicellular aggregates and macromolecules, a research method for multicellular aggregates, and a related product thereof.

(3) Beneficial Effects

The beneficial effects of the present invention are:
Firstly: The cellular physical information acquired by the present invention can be used to characterize the state of cells and/or multicellular aggregates before, during and after interaction with each other and with external factors, or including continuous states, enabling real-time observation of states. It can identify various states in a short time, at low cost and with high throughput, with an accuracy rate of over 98%; it can be applied to quickly determine the relationship between cells, between cells and multicellular aggregates, and between multicellular aggregates under different scenarios and conditions, and cell samples can be reused; the present invention is further limited to be implemented through a characterization system, which can achieve the above effects.

Secondly: The characterization system further defined by the present invention can realize the detection of cellular mechanical force through reflected light. Compared with existing cellular mechanical force detection devices, it has the characteristics of high throughput and low cost; compared with existing TFM and ordinary microcolumn arrays, the technical solution of the present invention gets rid of dependence on microscopes, greatly simplifying the operation process, because high-resolution imaging through microscopes is not required, and high-throughput monitoring of cells can be realized only by monitoring the intensity of reflected light, with low cost. The cellular mechanical force detection device converts cellular mechanical force into optical signals for detection based on microcolumn deformation caused by cellular mechanical force, with high accuracy and high sensitivity; the optical intensity has a linear correlation with the magnitude of cellular mechanical force, enabling qualitative and quantitative analysis of cellular mechanical force.

Thirdly: The characterization system further defined by the present invention optionally adds a magnetic metal reflection layer and magnetic material on the top of microcolumns, and by changing parameters such as coating composition characteristics, spacing, movement logic of microcolumns, it can switch between measuring cellular mechanical force or cellular hardness, or measure both cellular force and cellular hardness simultaneously, realizing more flexible and accurate comprehensive characterization of cellular physical characteristics.

Fourthly: The characterization system of the present invention has single-cell resolution: high resolution, enabling real-time monitoring of each cell, and can be combined with other single-cell analysis technologies to measure heterogeneity of cell responses to drugs; real-time monitoring: no need for fluorescence, avoiding phototoxic effects of lasers on cells, thus suitable for long-term monitoring and research on long-term responses of cells to drugs; high sensitivity: through reflection signals, the microcolumn deformation signal is amplified, increasing the sensitivity for deformation monitoring. Detection of micro/nanocolumn bending deformation generally relies on optical systems (such as microscopes), but the smaller the microcolumn size, the higher the requirements for precision and resolution of optical systems. For example, microcolumns with a width of 2 $\mu$m and a height of 6 $\mu$m require an objective lens of 20x or higher with a confocal system for effective observation. However, the present invention uses specular reflection principle to detect the attenuation of reflected light, actually amplifying the microcolumn deformation signal. Experimental verification shows that the same signal can be observed with a 5x objective lens. With a special reading system, effective detection of micro/nanocolumn deformation can be achieved without relying on high-magnification optical objectives, thereby greatly reducing system cost and effectively improving throughput.

Fifthly: The characterization system of the present invention can simulate the cellular microenvironment; it can simulate the composition and morphology of the extracellular matrix, coping with more abundant technical demand scenarios.

**Brief Description of Drawings**

FIG. 1 is a schematic structural diagram of a cellular mechanical force detection device in Example 1 of the present invention;

FIG. 2 is a scanning electron microscope (SEM) image of microcolumns (physical object) of a cellular mechanical force detection device in Example 1 of the present invention; wherein, a is a top view of the cellular mechanical force detection device, and b is a side view of the cellular mechanical force detection device;

FIG. 3 is a schematic structural diagram of a characterization system for cell/multicellular aggregate interaction related to Example 9 of the present invention;

FIG. 4 is a schematic structural diagram of a characterization system for cell/multicellular aggregate interaction related to Example 10 of the present invention;

FIG. 5 is a scanning electron microscope image of a microcolumn (polydimethylsiloxane) with a light reflection layer (gold) on the top; wherein, FIG. 5a is a scanning electron microscope image of the microcolumn; FIG. 5b is an element characterization diagram of the top region of the microcolumn; FIG. 5c is an element characterization diagram of the side region of the microcolumn (excluding the top region);

FIG. 6 is a picture of cells adhering to the top of microcolumns with cell adhesion substances (fibronectin) in Example 7 of the present application, wherein 6a is a fluorescence imaging diagram of cells adhering to a preset pattern composed of microcolumn groups with fibronectin on the top, and 6b is a cellular force distribution diagram calculated from light reflection signals measured on microcolumn groups with fibronectin on the top;

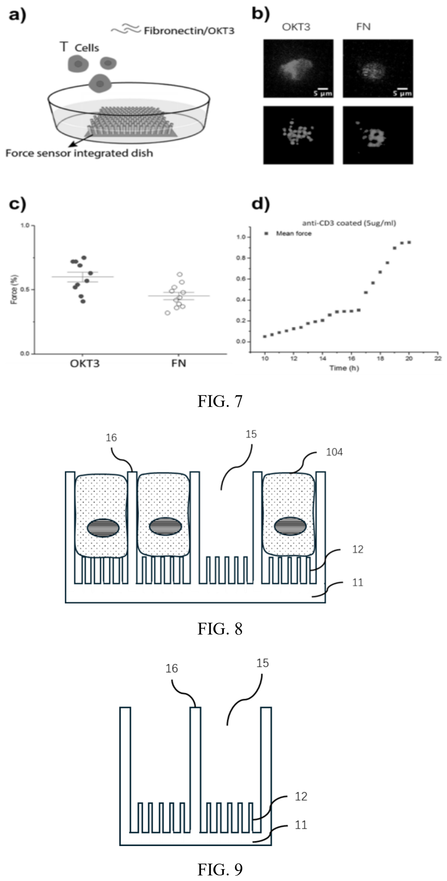

FIG. 7 is a picture related to experiments using OKT3 antibody (i.e., a substance interacting with cell surface receptors) or fibronectin (FN) as cell adhesion substances in a specific example of the present application, wherein 7a is a schematic diagram of experiments using OKT3 antibody as a cell adhesion substance; the upper two images in 7b are fluorescence imaging diagrams of cells adhering to the top of microcolumns with OKT3 antibody and fibronectin on the top respectively; the lower two images in 7b are cellular mechanical force distribution diagrams calculated from light reflection signals measured on microcolumns; 7c is a comparison diagram of mechanical force measured on surfaces coated with OKT3 antibody and fibronectin; 7d is a diagram of dynamic changes in cellular mechanical force of T cells after being planted on the surface (microcolumn top) coated with CD3 antibody;

FIG. 8 is a schematic structural diagram a of a cellular mechanical force detection device with a cell restriction mechanism;

FIG. 9 is a schematic structural diagram b of a cellular mechanical force detection device with a cell restriction mechanism;

FIG. 10 is a related picture of a cellular mechanical force detection device using a silicon film as a cell restriction mechanism in a specific example of the present invention, wherein a is a physical picture; b is a fluorescence microscope picture of the cellular mechanical force detection device using a silicon film as a cell restriction mechanism under light reflection; c is an enlarged view of b;

FIG. 11 is a fluorescence microscope image of monitoring cellular mechanical force by the characterization system of Example 11;

FIG. 12 is a schematic diagram of a cellular mechanical force detection system and its detection results provided by Example 12 of the present invention, wherein a is a schematic structural diagram of a characterization system for cell and/or multicellular aggregate interaction of Example 12; b is an image of light reflection signals of the cellular mechanical force detection device acquired by the light signal detecting device; c is a visualization effect diagram of mechanical force magnitude and distribution processed by the light signal analysis device;

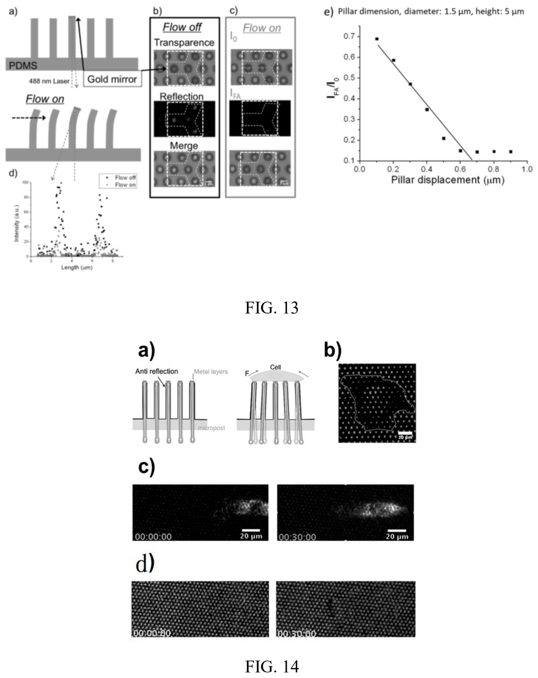

FIG. 13 is a schematic diagram of the relationship between cellular mechanical force and light reflection signals using fluid as an external force in a specific example of the present invention, a is a schematic structural diagram of the cellular mechanical force detection device in a microfluidic environment before and after fluid is turned on; b is a comparison diagram of bright field microscope images, reflected light signal distribution diagrams and their superimposed effect diagrams of microcolumns before fluid is turned on; wherein, the superimposed effect diagram refers to a diagram formed by superimposing the bright field microscope image of microcolumns and the reflected light signal distribution diagram; c is a comparison diagram of bright field microscope images, reflected light signal distribution diagrams and their superimposed effect diagrams of microcolumns after fluid is turned on; wherein, the superimposed effect diagram refers to a diagram formed by superimposing the bright field microscope image of microcolumns and the reflected light signal distribution diagram; d is the intensity value of light reflection signals before and after fluid is turned on; e is the linear interval between light reflection signal attenuation and microcolumn top translation;

FIG. 14 is a schematic diagram of a method for detecting cellular mechanical force acquired by the cellular mechanical force detection method of the present application in a specific example, a is a schematic structural diagram of microcolumns of the cellular mechanical force detection device before and after contact with cells; b is a reflected light signal distribution diagram acquired by the light signal detecting device; c is a monitoring diagram of cell migration process; d is a reflected light signal distribution diagram during cell migration process;

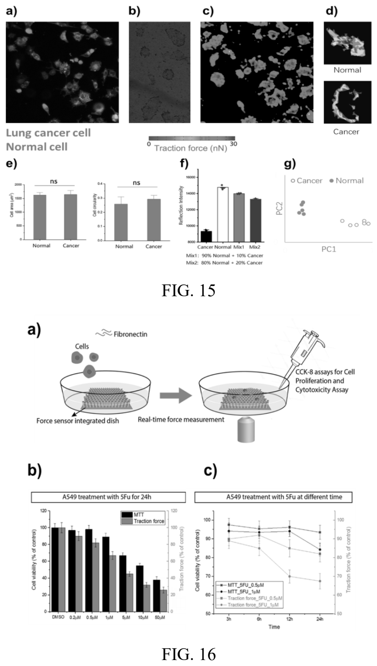

FIG. 15 is a diagram of cellular mechanical force information acquired by the cellular mechanical force detection method of the present application in a specific example, a is a fluorescence imaging diagram of a mixed system of healthy cells and lung non-small cell cancer cells; b is a distribution diagram of light reflection signals of the cellular mechanical force detection device acquired by the light signal detecting device; c is a visualization effect diagram of mechanical force magnitude and distribution processed by the light signal analysis device; d is an enlarged view of representative single-cell force distribution of healthy cells and lung non-small cell cancer cells in c; e is a comparison diagram of cell morphology between healthy cells and lung non-small cell cancer cells; f is a comparison diagram of reflected signal intensity between healthy cells, lung non-small cell cancer cells, and a mixture of these two cells in different proportions; g is a cluster analysis diagram obtained by structuring c and processing based on structured cellular physical information;

FIG. 16 is a schematic diagram of using cellular mechanical force information acquired by the cellular mechanical force detection method of the present application to monitor cell viability in a specific example, wherein a is a schematic diagram of the operation Process of the cell viability detection method; b is a comparison diagram of cell viability measured by MTT method and cell viability reflected by cellular mechanical force after A549 cells are treated with different doses of 5FU for 24h; c is a comparison diagram of cell viability measured by MTT method and cell viability reflected by cellular mechanical force after A549 cells are treated with different doses of 5FU for different times;

FIG. 17 is a schematic diagram of scalarization processing of displacement information at a certain point in Example 29 of the present invention;

FIG. 18 is a result diagram A of using the established cell feature model for identification of unknown cells or unknown cell phenotypes in an extended implementation of Example 30 of the present invention;

FIG. 19 is a result diagram B of using the established cell feature model for identification of unknown cells or unknown cell phenotypes in an extended implementation of Example 30 of the present invention;

FIG. 20 is an experimental result diagram of monitoring organoid attachment and response to drugs using mechanical force characterization in Example 35;

FIG. 21 is a fluorescence staining experimental result diagram of using mechanical force characterization for tumor spheroids in Example 36;

FIG. 22 is an experimental result diagram of using a restrictive structure to constrain the size and shape of cell spheroids in Example 36;

FIG. 23 is a diagram of fluorescence performance, cellular mechanical force imaging and quantification of T cell lines after activation by CD3 antibody coating on the device in Example 41 of the present invention;

FIG. 24 is a visualization effect of measuring mouse cardiomyocyte mechanical force by the cellular mechanical force measurement device in Example 53;

FIG. 25 is a diagram of changes in cellular mechanical force during differentiation of NIH3T3-L1 into adipocyte-like cells in Example 56;

FIG. 26 is a characterization diagram of stem cell-induced heart tissue by printing tropistic ECM on a chip in Example 58;

FIG. 27 is a diagram of fluorescence and histochemical staining characterization of stem cell-induced cartilage by printing tissue-specific ECM on a chip in Example 59;

FIG. 28 is a schematic diagram of a double-sided deformable lung tumor chip in a Example 60;

FIG. 29 is a cellular mechanical force imaging diagram of tumor tissue blocks and normal tissue blocks in Example 63;

FIG. 30 is a cellular mechanical force imaging diagram of tumor tissue blocks and normal tissue blocks after drug treatment in Example 63; and

FIG. 31 is a sandwich structure described in Example 36.

**Description of Reference Signs:**

1-Cellular mechanical force detection device; 2-Light signal generation device; 3-Light signal detecting device; 4-Light signal analysis device;

5-Beam splitter;

11-Base; 12-Microcolumn; 13-Light reflection layer; 15-Recessed space; 16-Restriction surface;

101-Objective lens; 102-Incident and reflected light; 103-Deformed microcolumn; 104-Cell; 105-Anti-reflection layer; 106-Substance with cell adhesion effect; 107-Substance with cell adhesion inhibition effect.

**Detailed Description of Embodiments**

In order to better explain the present invention, for easy understanding, the exemplary embodiments of the present invention will be described in more detail below. Although the following shows exemplary embodiments of the present invention, it should be understood that the present invention can be implemented in various forms and should not be limited by the embodiments set forth herein. On the contrary, these embodiments are provided to enable a more thorough understanding of the present invention and to fully convey the scope of the present invention to those skilled in the art.

**Embodiment 1**

This embodiment provides a method for characterizing cells and/or multicellular aggregates by acquiring cellular physical information of cells and/or multicellular aggregates. The cellular physical information includes cellular mechanical force and/or hardness at a specific point in the cell; the cellular mechanical force includes at least one of magnitude, direction, and frequency. The cellular physical information further includes cell morphology information. The cellular physical information includes changes in cellular mechanical force and/or hardness at the specific point over a certain time interval. Optionally, the cellular mechanical force and/or hardness in the cellular physical information are presented in a visualized form.

Optionally, the cellular physical information is acquired under cell confinement operations on cells and/or multicellular aggregates. Confinement enables ordered cultivation of a fixed number of cell groups in a restricted environment, allowing high-throughput monitoring of changes in their cellular mechanical forces, while enabling each cell group to contact other cell groups, facilitating observation of intercellular interactions.

"Identification" refers to the identification of known and unknown types.

In this embodiment, the cellular physical information is acquired based on the following interactions: interactions between cells and multicellular aggregates; interactions between cells; interactions between multicellular aggregates; interactions between cells and multicellular aggregates.

Specifically, it includes the following steps:
Disposing the first cell and/or multicellular aggregate and the second cell and/or multicellular aggregate in specific regions respectively, detecting and acquiring cellular physical information;

Alternatively,
After disposing the first cell and/or multicellular aggregate in a specific region, allowing the second cell and/or multicellular aggregate to interact with the first cell and/or multicellular aggregate, detecting and acquiring cellular physical information;

The first cell and/or multicellular aggregate and the second cell and/or multicellular aggregate each refer to one or more cells and/or multicellular aggregates. The interaction between the first cell and/or multicellular aggregate and the second cell and/or multicellular aggregate is characterized through the cellular physical information.

Optionally, the cell/multicellular aggregate characterization system of Embodiment 1 may be selected for characterization, where the specific region is located in the cell/multicellular aggregate characterization device system. Optionally, cells and/or multicellular aggregates may adhere to specific regions, optionally via limited adhesion.

Adhesion methods include: after placing cells and/or multicellular aggregates in a specific region (in other specific embodiments, a specific region of the characterization system), statically culturing for more than half an hour, and continuing to add culture medium until the cells and/or multicellular aggregates are completely attached to the specific region (characterization device).

Optionally, external stimuli are added before or during the detection and acquisition of cellular physical information. Optionally, the added external stimuli may include, but are not limited to: biological, chemical, physical stimuli, tropic guidance, and dynamic stimuli.

Drug stimulation is used to monitor and characterize the effect of drugs on cells and/or multicellular aggregates, as well as changes in interactions between cells and multicellular aggregates, between cells, between multicellular aggregates, and between cells and multicellular aggregates under drug stimulation. Furthermore, it clarifies the pharmacological effects and other properties of drugs. Beyond drugs, other chemical stimuli may include, but are not limited to: pH value, oxygen content, and sugar concentration.

Biological stimulation may include, but is not limited to:
Growth factors: Key signaling molecules for cell growth and proliferation. Adding growth factors can stimulate cells to promote proliferation, migration, or differentiation, thereby affecting cellular mechanical properties and interactions.

Extracellular matrix (ECM) components: Altering the composition or structure of the ECM (e.g., adjusting stiffness, fiber arrangement, or chemical composition) can directly influence cellular mechanical responses and behaviors.

Dynamic stimulation may include, but is not limited to:
Cyclic strain: Applying periodic strain stimulation (e.g., alternating tensile and compressive forces) to simulate dynamic changes in biological tissues during physiological processes, facilitating studies on cellular mechanical responses and adaptability.

Time-varying chemical environment: Periodically or stepwise altering the chemical environment (e.g., periodic changes in drug concentration or addition/removal of chemicals) to simulate dynamic cellular responses and adaptation under physiological or pathological conditions.

Tropic guidance may include, but is not limited to:
Forming biological gradients (e.g., structural or stiffness gradients of ECM) on substrates to guide directional cell growth or movement.

Scratching substrates in specific directions to guide cell growth along the scratch direction.

Physical stimulation may include, but is not limited to:
Temperature, magnetic induction, electrical stimulation, and flow field stimulation.

Mechanical forces: Applying mechanical stretching, compression, or other mechanical stimuli to directly regulate cell morphology and mechanical responses, including but not limited to altering cell shape and intracellular force distribution.

Fluid mechanical stimulation: Applying fluid mechanical stimuli (e.g., shear stress, flow field changes) to simulate the physiological environment of cells in blood or tissue fluids, thereby regulating cellular physiological activity and mechanical properties.

External stimuli can amplify differences in cellular mechanical force information between different cell types, facilitating easier identification and improving recognition efficiency and accuracy. When using characterization devices containing microcolumns to detect mechanical force and hardness, cellular physical information is amplified by adjusting the hardness of microcolumns.

Adhesion of cells and/or multicellular aggregates can be stabilized by adding cell culture medium for continued cultivation, and/or by using adhesive substances to attach cells and/or multicellular aggregates.

In this embodiment, cellular physical information of cells/multicellular aggregates is acquired to enable identification of interactions between cells and/or multicellular aggregates. By acquiring cellular physical information, this embodiment enables rapid, high-throughput identification of differences between cells/multicellular aggregates, applicable to cellular/multicellular aggregate responses to drugs.

In this embodiment, cellular mechanical forces can be monitored and acquired using microforce sensors, microrheometers, or cellular mechanical force detection devices. These devices can measure the magnitude, direction, and frequency of forces exerted by cells on substrates or other cells. Changes in cellular mechanical forces can be tracked by measuring at specific time intervals.

Hardness monitoring: The hardness of cells or multicellular aggregates can be measured using nanoindentation, force-distance curve techniques, etc. These techniques enable quantitative assessment of cell or aggregate hardness to understand changes under external forces.

Cell morphology and spatial distribution monitoring: Cell morphology and spatial distribution can be acquired using imaging techniques such as microscopes, confocal microscopes, and atomic force microscopes. These techniques allow observation of cell morphological features, internal structures, and spatial distribution of cell aggregates.

In this embodiment, cellular physical information can be acquired through a characterization system, which includes:

A base; and

A microcolumn array composed of one or more microcolumns disposed on the base, which can deform under cellular mechanical force and/or magnetic force, with a light reflection layer on the microcolumns.

Optionally, a light reflection layer is disposed at the end of the microcolumn away from the base; the base has a light-transmissive portion; the column body of the microcolumn has a light-transmissive portion; optionally, the surface of the microcolumn and/or the base has an anti-reflection layer.

Optionally, it is further acquired through a light signal emitting device and a light signal detecting device. Light emitted by the light signal emitting device irradiates the light reflection layer through an incident optical path, and light reflected by the light reflection layer enters the light signal detecting device through a reflected optical path.

Optionally, the optical intensity acquired by the light signal detecting device is analyzed to obtain cellular physical information. Optionally, the optical intensity acquired by the light signal detecting device has a linear correlation with the magnitude of cellular mechanical force, enabling qualitative and quantitative analysis for different cell typing.

Optionally, the characterization device can contain liquids; if the liquid is cell culture medium, cells and/or multicellular aggregates are allowed to adhere and/or continue cultivation. Optionally, cells and/or multicellular aggregates are adhered to microcolumns via adhesive substances disposed on the microcolumns.

In methods for acquiring cellular physical information through characterization devices, multicellular aggregates can be combined with the characterization device in multiple ways. In other specific embodiments, two specific combination methods are provided:

First combination method: Disposing culture medium on microcolumns of the cellular mechanical force detection device, transplanting cells into the culture medium on the microcolumns, and culturing to obtain multicellular aggregates. In other embodiments, this combination allows real-time monitoring of cell culture processes when cellular physical information is output in a visualized form, applicable to studying effects of chemical, biological, and physical external stimuli (e.g., culture medium, drugs) on cell growth.

Second combination method: Directly adhering pre-cultured multicellular aggregates to microcolumns of the cellular mechanical force detection device for detection.

This embodiment further provides a method for typing and identifying cells and/or multicellular aggregates using the cellular physical information described in any of the above items. The typing and identification include: types, states, behaviors, spatial omics characteristics, differentiation directions, and stress responses of cells and/or multicellular aggregates. Optionally, the typing and identification include selectively sorting specific cells or tissues through the cellular physical information.

Optionally, identification is performed using a cell identification device, which includes an information acquisition unit, a preprocessing unit, a learning unit, and an identification unit:
The information acquisition unit is used to acquire cellular physical information of cells and/or multicellular aggregates.

The preprocessing unit is used to preprocess cellular physical information to form structured cellular physical information, which includes the number of cells, the number of cell features, and feature information of each cell feature.

The learning unit is used to establish a cell feature model using supervised, unsupervised, or semi-supervised machine learning with structured cellular physical information as input data.

The identification unit is used to apply the cell feature model to classification or clustering of cells and/or multicellular aggregates to achiving typing and identification of cells and/or multicellular aggregates.

"Identification" in the present invention refers to identifying cells and/or multicellular aggregates of known and unknown types. "Typing" in the present invention refers to classifying cells and/or multicellular aggregates based on differences in properties including but not limited to type, state, behavior, and spatial omics characteristics caused by different factors. Cells with identical or similar cellular physical information are clustered into types, which can further form different categories.

The present invention enables rapid, high-throughput identification of differences between cells/multicellular aggregates by acquiring cellular physical information, applicable to responses of cells/multicellular aggregates to drugs. Typing of cells and/or multicellular aggregates refers to classifying cells and/or multicellular aggregates based on differences in properties including but not limited to type, state, behavior, and spatial omics characteristics caused by different factors.

**Embodiment 2**

This embodiment provides a method for characterizing cells and/or multicellular aggregates, which differs from Embodiment 1 in that it acquires cellular physical information based on multicellular aggregates at different growth time points and/or different regions within multicellular aggregates.

The method for acquiring cellular physical information may include, but is not limited to: placing multicellular aggregates in a specific region and adding cell culture medium to enable stable adhesion of multicellular aggregates, facilitating the measurement of cellular physical information.

In other specific embodiments, the characterization system described in Embodiment 1 may be used to acquire the cellular physical information. In other specific embodiments, the characterization system can contain cell culture medium; adding cell culture medium enables the acquisition of tissue cellular mechanical force while culturing the tissue on the cellular mechanical force detection device (characterization device) without removing the tissue from the cellular mechanical force device.

In other specific embodiments, the liquid level of the cell culture medium is higher than the top of the microcolumns. In other specific embodiments, the method for acquiring tissue cellular physical information includes: placing the tissue on the characterization system, adding cell culture medium to at least bring the tissue into contact with the cell culture medium, statically culturing for more than half an hour, continuing to add cell culture medium until the tissue is completely attached to the microcolumns, and then real-time measuring and acquiring the cellular mechanical force of the tissue. Culturing for more than half an hour facilitates better fixation of the tissue with the microcolumns. Optionally, after the tissue is completely attached to the cellular mechanical force device, external stimuli are added to measure changes in cellular mechanical force of the tissue under external stimuli. In other specific embodiments, multicellular aggregates (which may be tissues) are adhered to the microcolumns through adhesive substances disposed on the microcolumns. In other specific embodiments, the multicellular aggregate refers to a tissue, and the tissue may optionally be a tissue section of 150-200 mm.

Through the method of this embodiment:
Optionally, the tissue includes active tissue, organoids, and in vitro organs.

Optionally, external stimuli are applied to the multicellular aggregate before, during, and after measuring and acquiring cellular physical information.

Optionally, after the tissue is completely attached to the cellular mechanical force device, external stimuli are added to measure changes in cellular mechanical force of the tissue under external stimuli.

External stimuli can amplify differences in cellular physical information between different regions within multicellular aggregates and between different types of multicellular aggregates, facilitating easier identification and improving recognition efficiency and accuracy. When using a cellular mechanical force detection device containing microcolumns to detect mechanical force and hardness, cellular physical information is amplified by changing the hardness of the microcolumns. Specific external stimuli are as described in Embodiment 1.

Different regions within multicellular aggregates include, but are not limited to: tumor regions and non-tumor regions in tissues; after characterization, tumor and non-tumor regions in tissues can be identified. They also include, but are not limited to: **Peripheral region:** Located at the outer edge of the multicellular aggregate, in contact with the surrounding environment, and may be exposed to the medium or culture medium.

**Central region:** The central part of the multicellular aggregate, usually denser due to increased cell density, and may have different cellular tissue structures.

**Marginal region:** The region between the peripheral and central regions, usually exhibiting different characteristics from the peripheral and central regions.

**Core region:** The core part of the multicellular aggregate, which may be the most densely cellular region or contain specific cell types or groups.

**Surface region:** The external surface region of the multicellular aggregate, which may be directly exposed to the external medium and in direct contact with the surrounding environment.

**Functional region:** A region with specific functions or activities within the multicellular aggregate, such as metabolically active regions, secretory regions, or differentiation regions.

**Gradient region:** A gradient region within the multicellular aggregate, which may contain gradient distributions of signaling molecules or nutrients.

**Microenvironment region:** The microenvironment within the multicellular aggregate, which may be influenced by cell secretions, cell-cell interactions, or matrix components, and has an important impact on cell behavior and function.

These different regions may exhibit differences in cell proliferation, differentiation, signal transduction, metabolism, and cell interactions, and have an important impact on the overall behavior and function of the cell aggregate. This embodiment can characterize, type, and identify not only the above regions but also unknown regions. This embodiment can form structured information from the cellular information of the state of multicellular aggregates (tissues) of different types. Different tissues can be divided into: two or more different tissues, one tissue in different states formed at different times or under external stimuli, and two or more different regions in one tissue. Two or more different tissues: may be pathological tissues and normal tissues.

One tissue in different states formed at different times or under external stimuli: may be tissues in different states corresponding to different times under dynamic changes after adding drugs, or changes in tissue state during culture with cell culture medium.

Two or more different regions in one tissue: may be a tissue with tumor regions and non-tumor regions.

This embodiment can deform microcolumns through the cellular mechanical force of multicellular aggregates (tissues), thereby converting and amplifying cellular mechanical force into light reflection signals, enabling accurate identification of tissue cellular mechanical force. It can be applied to real-time monitoring of tissue during changes, and even if the tissue has only slight changes during culture, changes in its tissue cellular mechanical force can be acquired, endowing each changed tissue with a mechanical fingerprint. It can also pierce cells with microcolumns through magnetic force to measure hardness, achieving characteristic, accurate, and rapid identification.

This embodiment can be used for rapid, direct, non-destructive, and real-time measurement of cellular mechanical force of entire living tissues and each cell in living tissues; cellular mechanical force is measured through contact between cells in the tissue and microcolumns. In addition, since each cell type has different cellular mechanical strength, measuring the mechanical distribution of the entire tissue using the characterization system can identify blocks of different cells in the tissue, such as identifying tumor and non-tumor regions. Furthermore, when measuring the cellular mechanical force of tissues, drugs can be directly added for treatment, and changes in cellular mechanical force of cells in the tissue can be monitored in real-time to determine whether the drug is effective, thereby achieving precise drug screening with an accuracy rate of over 98%.

Except for the above differences, this embodiment is the same as Embodiment 1 and will not be repeated here.

This embodiment provides a method for rapid, direct, non-destructive, and real-time measurement of mechanical force of entire living tissues and individual cells within living tissues. Cellular mechanical force can be measured through contact between cells in the tissue and microcolumns. In addition, measuring the mechanical distribution of the entire tissue using the characterization system can identify blocks of different cells, such as tumor and non-tumor regions. Meanwhile, this method can directly introduce drug treatment while measuring tissue cellular mechanical force and monitor changes in cellular mechanical force in real-time to evaluate drug efficacy, achieving precise drug screening with an accuracy rate of over 98%.

In addition, this method can realize real-time monitoring of cellular mechanical force during tissue culture and dynamic changes, quickly measure cellular mechanical force of the entire living tissue, identify tumor and non-tumor regions using cellular mechanical force strength, and monitor changes in mechanical force of tumor cells in real-time after drug treatment to determine whether the drug can effectively inhibit tumor cell growth. This method converts tissue cellular mechanical force information into structured information to realize the determination and identification of different tissue states. Correspondingly, it also provides a characterization system and identification system for tissue states, as well as use in tissue state identification and methods for evaluating drug efficacy against tumors.

**Embodiment 3**

This embodiment provides a method for characterizing cells and/or multicellular aggregates, which differs from Embodiment 1 in that it is based on cellular physical information obtained from the interaction of substances with cells and/or multicellular aggregates.

The method for obtaining cellular physical information includes the following steps:

Allowing the multicellular aggregate substance to interact with the cells and/or multicellular aggregates;

Detecting and acquiring cellular physical information of the cells and/or multicellular aggregates.

Optionally, add culture medium to at least bring the cells and/or multicellular aggregates into contact with the cell culture medium, statically culture for more than half an hour, continue adding cell culture medium until the cells and/or multicellular aggregates are completely attached to a specific region, then add the interacting substance and continue culturing, while measuring and acquiring cellular physical information. Culturing for more than half an hour facilitates better fixation of cells/multicellular aggregates with the microcolumns.

Optionally, external stimuli are added before or during the detection and acquisition of cellular physical information.

Except for the above differences, this embodiment is the same as Embodiment 1 and will not be repeated here.

The substances include bioactive macromolecules, chemical substances, bioactive substances, and inactivated biological substances; the bioactive macromolecules include proteins, polypeptides, polysaccharides, and lipids.

This embodiment provides a cellular mechanical force detection device, a cell/cell aggregate characterization system, or a method for detecting cellular mechanical force as described in any of the above embodiments to obtain cellular mechanical force information of cells/multicellular aggregates. Specifically, it includes the following steps: placing the cells/multicellular aggregates on the cellular mechanical force detection device, adding cell culture medium to at least bring the cells/multicellular aggregates into contact with the cell culture medium, statically culturing for more than half an hour, continuing to add cell culture medium until the cells/multicellular aggregates are completely attached to the microcolumns, then measuring and acquiring the cellular mechanical force of the cells/multicellular aggregates. In some specific embodiments, external factors are added to detect dynamic changes in cellular mechanical force of cells/multicellular aggregates under external factors; in some specific embodiments, external factors can be added before or during the detection of cellular mechanical force. In some specific embodiments, substances can be produced by the cells/multicellular aggregates or added externally.

This embodiment also provides a method for obtaining visualized cellular information including cells and/or multicellular aggregates before, during, and after the interaction between the substance and the cells/multicellular aggregates through the cellular mechanical force detection device, including visualized information such as the magnitude and distribution of changes in cellular mechanical force. The visualized information is used for visual identification, real-time monitoring, and characterization of the interaction between the substance to be tested and the cells/multicellular aggregates.

In other embodiments, the cellular mechanical force information further includes the direction of the cellular mechanical force at the specific point. In other embodiments, the cellular mechanical force information further includes changes in the magnitude or direction of the cellular mechanical force at the specific point over a certain time interval. In other embodiments, the cellular information further includes cell morphology information. In other embodiments, cellular mechanical force information is obtained by the method of Embodiment 18.

Based on the cellular mechanical force detection device of the present invention, not only can qualitative analysis be performed by visual distinction with the naked eye, but also more intuitive and accurate identification (quantitative and qualitative analysis) of the state of cells/multicellular aggregates can be achieved based on the measured cellular mechanical characteristics, and it is confirmed that using the cellular force field as a marker can better distinguish types.

The present invention also provides a system and method for identifying substances and cells/multicellular aggregates, which converts cellular information including cellular mechanical force information from the action of substances on cells/multicellular aggregates into structured information, enabling rapid and automatic determination and identification of the interaction between the substance to be tested and the cells/multicellular aggregates.

Correspondingly, the present invention also provides use of the cell/multicellular aggregate characterization and identification system and method in related methods and products requiring characterization of interactions between cells/multicellular aggregates.

It can be applied in different scenarios and conditions to quickly determine the mechanism of action between substances and cells/multicellular aggregates, enabling monitoring of the effects of substances on cells, changes in substances inside and outside cells, or monitoring of the effects of substances on cells and changes in cell states, with reusable cell samples.

Furthermore, the cellular mechanical force detection device of the present invention can detect cellular mechanical forces of multi-layered cells, including tumor aggregates, making it applicable in drug screening, regenerative medicine, gene editing, precision medicine, organ development, and disease modeling requiring multi-cell analysis.

**Embodiment 4**

This embodiment provides a method for characterizing cells and/or multicellular aggregates, which differs from Embodiment 3 in that cellular physical information can also be obtained based on the action of other physical, biological, or chemical stimuli on cells and/or multicellular aggregates.

The method includes:

Placing cells and/or multicellular aggregates in a specific region (in other embodiments, in the characterization system);

Monitoring subtle changes in cellular mechanical force during culture or under external factors, enabling measurement of state changes in multicellular aggregates or cells and characterization of stress responses of cells and/or multicellular aggregates under external factors.

Optionally, external stimuli include drugs, mechanical force, biochemical factors, electric fields, tropic guidance, dynamic stimuli, and combinations of one or more stimuli in flow fields acting on the culture.

In an optional solution, before, during, or after using the characterization system, the sample to be tested can be subjected to different types and intensities of stimuli (mechanical, electrical, optical, etc.), and operations (labeling, solidification, ablation, cutting, extraction, sorting, etc.) can be performed on specific positions or regions of the sample; it can be combined with other characterization methods (protein staining, histochemical staining, single-cell sequencing, etc.) for comparative analysis. Optionally, it further includes an action mechanism acting on the characterization system or cells, where the action mechanism is one or a combination of microfluidics, microneedles, and lasers. Defining appropriate microcolumn height, spacing between adjacent microcolumns, and column surface size within the above ranges can keep cells/multicellular aggregates in a more stable state in the substance and cell/multicellular aggregate characterization system.

The method of this embodiment can be used to characterize cell viability, adhesion, migration, activation, differentiation, and apoptosis.

In other specific embodiments, stimuli are used to induce stress in cells and/or multicellular aggregates, including physical stimuli, biochemical stimuli, and stimuli between small and large molecules on cells and/or multicellular aggregates.

The obtained cellular physical information includes cellular mechanical force and dynamic changes of cells and/or multicellular aggregates before, during, and after stress.

Cells perceive and respond to external stimuli through different mechanisms, including matrix stiffness, shear stress, and mechanical tension. The present invention can first fix and attach cells and/or multicellular aggregates at specific positions, then apply stimuli, or continue adding other cells and/or multicellular aggregates onto the attached cells and/or multicellular aggregates, enabling real-time monitoring of changes in cellular physical information of the attached cells and/or multicellular aggregates, and real-time monitoring of interactions between cells, between multicellular aggregates, or between cells and multicellular aggregates.

In an optional solution, before, during, or after characterization using the characterization system, the sample to be tested can be subjected to different types and intensities of stimuli (mechanical, electrical, optical, etc.), and operations (labeling, solidification, ablation, cutting, extraction, sorting, etc.) can be performed on specific positions or regions of the sample; it can be combined with other characterization methods (protein staining, histochemical staining, single-cell sequencing, etc.) for comparative analysis.

Except for the above differences, other contents of this embodiment are the same as Embodiment 1 and will not be repeated here.

**Embodiment 5**

This embodiment provides a method for characterizing cells and/or multicellular aggregates, which differs from Embodiment 1 in that it is based on cellular physical information during the growth of in vitro organs and/or related cell models; the cellular physical information includes cellular physical information of cells and/or any region during the seeding and culture of organ-related cells and/or tissue cultures.

In other specific embodiments, the cellular physical information is used to characterize cells, cell clusters, or organ tissues in any region during the growth of in vitro organs and/or related cell models.

In other specific embodiments, a mixture of cells and/or tissues with ECM is seeded.

In other specific embodiments, seeding methods include 3D printing and/or microfluidic methods to control the distribution and flow of cells and biological materials for seeding.

In other specific embodiments, organ-related cells and/or tissue cultures are seeded on a characterization device to prepare in vitro organ and cell models.

In other specific embodiments, the specific method includes:

S1. Collect microenvironmental parameters of the corresponding tissue under physiological and pathological states, and prepare a corresponding in vitro organ chip from the characterization device based on the parameters (i.e., the characterization device can be customized as an in vitro organ chip);

S2. Seed cultures containing the organ-related cells and/or tissues on the in vitro organ chip. Seeding can be performed on the in vitro organ chip by 3D printing.

S3. Optionally add physical and biochemical stimuli, including one or a combination of drugs, mechanical force, biochemical factors, electric fields, and flow fields, acting on the culture;

S4. Obtain changes in cellular mechanical force of each cell and tissue through the in vitro organ chip to characterize each cell and organ tissue.

Optionally, it further includes step S4: selectively sorting specific cells or tissues through the characterization.

The identification unit in the cell identification device is used to apply the cell feature model to classification or clustering of organs, cell growth states, or responses to external stimuli, enabling identification and characterization of organs and cells in corresponding states at each moment.

The characterization system of the present invention forms structured information from organ cellular information in corresponding states at each moment, enabling automatic identification of unknown cell states by analyzing cellular information in unknown states.

The in vitro organ chip can simulate the structural microenvironment of human organs, accurately approximating the real organ environment; meanwhile, it can monitor the behavior of each cell in real-time for extended periods, enabling organ characterization and application in establishing in vitro organs and related cell models for various research projects requiring such models.

In other specific embodiments, the in vitro organ chip is applied in screening drugs for organ treatment and studying organ models under physiological and pathological states.

The in vitro organ chip in this embodiment can not only simulate the structural microenvironment of in vivo organs but also characterize them by real-time detection of cellular mechanical force of each cell and organ tissue; it can monitor subtle changes in cellular mechanical force during culture or under external stimuli, enabling measurement of organ tissue or cell changes during culture or under external stimuli. External stimuli include one or a combination of drugs, mechanical force, biochemical factors, electric fields, and flow fields acting on the culture. Unlike traditional life science characterization methods, the characterization method of the present invention is non-invasive, label-free, and enables long-term real-time monitoring of living cells or tissues with single-cell resolution, better approximating the real organ environment.

Optionally, the chip body is provided with microcolumns of corresponding stiffness and length according to the organ; microcolumns of different stiffness and length can be customized for different organs to simulate a more realistic organ environment. For example, as a heart chip, it can create a cardiac microenvironment on a microchip, including mechanical contraction, molecular transport, electrical activity, and biochemical stimuli, with high customizability. It can simulate the complex structure and function of the heart in a small space, including myocardial cells, vascular endothelial cells, myocardial extracellular matrix, and other tissue components, thereby more accurately simulating cardiac function.

Optionally, the microcolumn surface is provided with ECM; optionally, a mixture of organ-related cells and/or tissues with ECM is seeded on the microcolumns; optionally, the microcolumn surface has a tropic ECM coating; optionally, the seeding method includes 3D printing. For better controllability and reproducibility, 3D printing and microfluidic technologies can be used to precisely control the distribution and flow of cells and biological materials, simulate blood flow in organs such as the heart, and standardize microenvironmental parameters to improve experimental reproducibility and data comparability. Optionally, if the organ is the heart, the cells include one or a combination of myocardial cells, smooth muscle cells, vascular endothelial cells, fibroblasts, stem cells, and immune cells.

Optionally, it further includes an electrode device, where the microcolumns are made of conductive material, and the electrode device acts on the microcolumns.

Optionally, it further includes a mechanical simulation device, which is a mechanical stretching device for stretching the chip body, or an inflatable deformable flexible membrane disposed at the bottom of the microcolumns; the upper end of the flexible membrane is connected to the microcolumns, and the lower end is connected to the base, or the base is a flexible membrane.

External stimuli are applied to the seeded region of the in vitro organ before, during, and after measuring and acquiring cellular physical information.

External stimuli can amplify differences in cellular physical information between different regions within multicellular aggregates, different cell types, and multicellular aggregates, facilitating easier identification and improving identification efficiency and accuracy. When using a cellular mechanical force detection device containing microcolumns to detect mechanical force and hardness, cellular physical information is amplified by changing the stiffness of the microcolumns. Specific external stimuli are as described in Embodiment 1.

Optionally, if the organ is the heart, the cells include one or a combination of myocardial cells, smooth muscle cells, vascular endothelial cells, fibroblasts, stem cells, and immune cells.

The in vitro organ chip provided in this embodiment is applied in screening drugs for organ treatment and studying organ models under physiological and pathological states.

The method and in vitro organ chip in this embodiment have better characterization capabilities by detecting cellular mechanical force, being non-invasive, label-free, enabling long-term real-time monitoring of living cells or tissues with single-cell resolution; the in vitro organ chip can serve as an important technical means through cellular mechanomics to address limitations of existing cell and animal models, thereby promoting further development and application of organ research such as the heart.

Unlike traditional life science characterization methods, the in vitro organ chip of the present invention better approximates the real organ environment. The heart chip can create a cardiac microenvironment on a microchip, including mechanical contraction, molecular transport, electrical activity, and biochemical stimuli, with high customizability. It can simulate the complex structure and function of the heart in a small space, including myocardial cells, vascular endothelial cells, myocardial extracellular matrix, and other tissue components, thereby more accurately simulating cardiac function.

For better controllability and reproducibility, 3D printing and microfluidic technologies can be used to precisely control the distribution and flow of cells and biological materials, simulate blood flow in organs such as the heart, and standardize microenvironmental conditions to improve reproducibility and data comparability. It is more economical, efficient, safe, and ethical compared to animal experiments or clinical trials, saving time and costs while being safer and more ethical.

By real-time monitoring of cellular mechanical force of each cell, the in vitro organ chip of the present invention enables characterization of organs and each cell in each state in a short time, low cost, and high throughput, thereby achieving accurate identification and long-term monitoring of the effects of external stimuli such as drugs on organs and cells at each moment, with an accuracy rate of over 98% for drug screening and over 98% for identifying different states under other external stimuli.

Except for the above differences, other contents of this embodiment are the same as Embodiment 1 and will not be repeated here.

**Embodiment 6**

This embodiment provides a method for identifying and typing cells and/or multicellular aggregates: It can also perform typing and identification through the cellular physical information obtained in Embodiments 1 to 5, including:

Through the characterization of the interaction between substances and cells or multicellular aggregates, determine whether the cells or multicellular aggregates are successfully transfected, including one or a combination of two or more of proteins and/or polypeptides, semi-solid medium, antibodies, or secondary antibodies;

Through the characterization of the interaction between bioactive substances and cells or multicellular aggregates, determine and screen high-yield cells or multicellular aggregates of bioactive substances;

By obtaining the cellular mechanical force of cells/multicellular aggregates, the system can monitor the impact of genetic engineering on cells, including: detecting small clone clusters or single cells near large clones;

By monitoring cell viability and state, help establish the optimal dose-response curve (kill curve) to determine the minimum effective concentration to kill non-resistant cells; optionally, monitor cells in real-time during drug screening to adjust experimental protocols in a timely manner.

By obtaining cellular mechanical force information of adipocytes including growth and differentiation processes, monitor the growth and differentiation of adipocytes; optionally, used to real-time monitor the transition process of white fat, beige fat, and brown fat.

The identification method includes the following steps: Obtain cellular physical information of cells/multicellular aggregates through a cellular mechanical force detection device to realize the identification of the interaction between substances and cells/multicellular aggregates; the cellular physical information includes cellular mechanical force information; the present invention can realize rapid and high-throughput identification of differences between cells/multicellular aggregates by obtaining cellular mechanical force, which can be applied to the response of cells/multicellular aggregates to substances.

Except for the above differences, this embodiment is the same as Embodiment 1, and will not be repeated here.

**Notes to be noted in any of the above embodiments:**

This embodiment obtains cellular physical information before, during, and after the action.

The cellular physical information in this embodiment can be measured in real-time, or at specific times or specific time intervals, and the cellular physical information can be continuous or intermittent.

This embodiment can characterize cells and/or multicellular aggregates at different growth times and under external stimuli, and thus perform typing and identification of known or unknown types.

This embodiment is not limited to characterization at different growth times and under a specific external factor, but can be characterization of cells and/or multicellular aggregates under the combined action of various factors.

Through the characterization, this embodiment can be used for clustering and classification of cells and/or multicellular aggregates at any time before, during, and after the action of substances on cells and/or multicellular aggregates, especially unknown classifications.

Any of the above embodiments can use the cellular mechanical force detection device and characterization system in any of the following examples as the cellular physical information characterization device or characterization system to obtain and analyze cellular physical information.

**Example 1: Cellular Mechanical Force Detection Device**

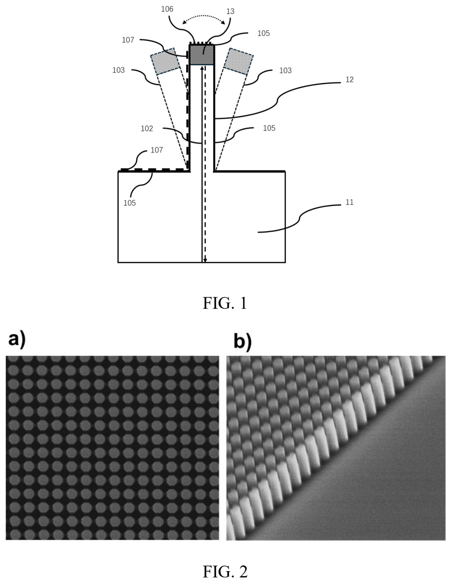

Please refer to FIG. 1, which is a structural schematic diagram a of a cellular mechanical force detection device. The cellular mechanical force detection device shown in the figure includes a light-transmitting base 11 (in other specific examples, the base is provided with a light-transmitting part, that is: it does not have to be a fully light-transmitting base, and can be provided with an opaque part) and microcolumns 12 arranged on the base 11, which can be deformed under the action of cellular mechanical force. The top of the microcolumn 12 is coated with a light reflection layer 13, and the thickness of the light reflection layer 13 is 5nm (in other embodiments, the thickness of the light reflection layer 13 can be between 5 nm and 20 nm-the thickness of the coating is related to the coating material. Under the premise of applying the same coating material, the selection of coating thickness should be limited to ensuring light transmission effect, microcolumn stability, and ensuring connection with the microcolumn does not fall off). The column of the microcolumn 12 can transmit light, such as the incident and reflected light 102 shown in FIG. 1, where the incident light is represented by a solid arrow, and the reflected light is represented by a dashed arrow. The arrow clusters in opposite directions in the figure represent incident light and reflected light. (Note: The term "coating" is used in this example, only indicating that the light reflection layer 13 in this example can be prepared by a coating process, and does not limit the light reflection layer 13 to be prepared by a coating process.) In other specific examples, as shown in FIGS. 1 and 14, the opaque part of the base is formed by the arrangement including but not limited to the anti-reflection layer 105.

Please refer to FIG. 2, which is a scanning electron microscope (SEM) image of the microcolumn 12 (physical object) of the cellular mechanical force detection device of this example, where a is a top view of the cellular mechanical force detection device, and b is a side view of the cellular mechanical force detection device. It can be seen from FIG. 2 that the microstructure of the microcolumns of the cellular mechanical force detection device is neat and uniform, and the size is controllable. Compared with existing cellular mechanical force detection devices, the mechanical values measured by the cellular mechanical force detection device of this example are more accurate.

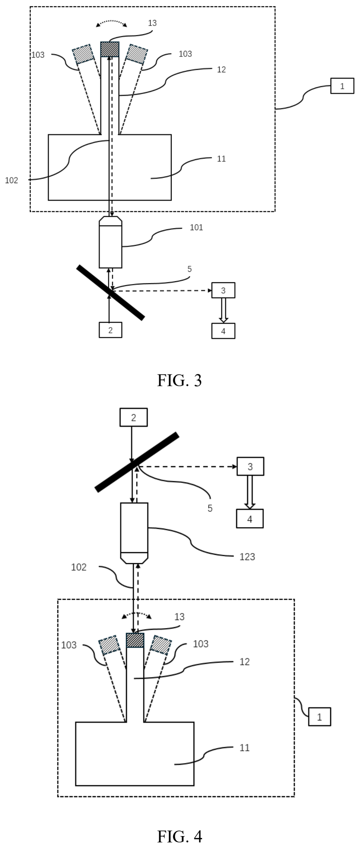

When the cellular mechanical force detection device 1 described in this embodiment is put into use, the number of microcolumns 12 will be more than one. Please refer to FIG. 3, which is a structural schematic diagram of a characterization system for cell/multicellular aggregate interaction related to the ninth example of the present invention; FIG. 3 can be used for understanding this example. The system shown in FIG. 3 includes, in addition to the cellular mechanical force detection device 1 described in this example, an optical signal generating device 2 with a light source and an optical signal detecting device 3 arranged below the base 11. The light emitted by the light source irradiates the light reflection layer of the microcolumn 12 from the light-transmitting base 11 of the cellular mechanical force detection device 1 through the incident optical path; the optical signal detecting device 3 is used to detect the light reflected from the light reflection layer 13 on the top of the microcolumn. The light reflected by the light reflection layer 13 passes through the reflection optical path and acts on the beam splitter 5 before entering the optical signal detecting device 3. After obtaining the reflected light signal, an optical signal analysis device 4 can compare and analyze the reflected light before and after the cellular mechanical force action between the cellular mechanical force detection device 1 and the cell to be tested, so as to obtain cellular mechanical force information. When the microcolumn 12 is not under force, the microcolumn should remain upright, so as to reflect the detection light to the maximum extent; when the microcolumn 12 is in contact with the cell, under the action of cellular mechanical force, the microcolumn 12 deforms (but not limited to bending and swinging), resulting in a decrease in light reflection level. Therefore, the greater the cellular mechanical force, the smaller the obtained light reflection signal, so the magnitude of the cellular mechanical force at that point can be easily inferred by observing the intensity of the light reflection signal.

In addition, the measurement light source in the technical solution of this example can use an infrared laser with a certain intensity. The microcolumn measurement in the traditional technical solution requires taking high-resolution images, and in this process, the use of laser is likely to cause cell phototoxicity or sample fluorescence quenching. However, in this technical solution, if only the reflection signal is to be measured, the influence of the infrared laser within a certain light intensity on the cells can be basically ignored, so it is suitable for long-term monitoring of cells.

## Example 2: Cellular Mechanical Force Detection Device

The difference from the first example is that the microcolumn 12 not only has a light reflection layer 13 on the top end surface, but also has a light reflection layer 13 on the upper half of the column surface (that is, the curved surface connecting the two end surfaces of the column). In fact, in other embodiments, except that the scheme of arranging the light reflection layer 13 on the lower half of the side column surface of the microcolumn 12 is not adopted due to poor actual effect, as long as the light reflection layer 13 is arranged on the upper half of the side surface of the microcolumn 12, the detection effect desired by the present invention can basically be achieved. That is to say, in some other embodiments, the light reflection layer 13 can even be laid on any local position of the upper half side column surface or the local position of the top, and does not necessarily have to be covered with the entire upper half column surface or the entire top end surface, all of which can achieve the expected purpose, although there may be differences in the obtained data and the effect of later calculations.

In addition, the definitions of "column surface" and "end surface" of the microcolumn appear in the first and second examples of the present invention, that is to say, the independent column body we usually understand should have two end surfaces and a curved surface (column surface) connecting the two end surfaces, but the microcolumn in the present invention has only one end surface, that is, the top end surface, due to the existence of the base, and the other end is fixedly connected to the base or integrally formed with the base. However, in other embodiments, the top end surface may be a curved surface smoothly connected with the column surface as a whole, and does not necessarily have an intersection line or obvious boundary as shown in the first or second example. In this case, the installation position of the light reflection layer 13 will also be understood as the upper half of the column body, and cannot be limited to "end surface" or "column surface".

## Example 3: Cellular Mechanical Force Detection Device

Please refer to FIG. 4, which is a structural schematic diagram of a characterization system for cells/multicellular aggregates in the tenth example of the present invention, used to illustrate the cellular mechanical force detection device 1 in this example. The difference between this example and the first and second examples is that there is no requirement for the light transmission performance of the base 11 and the microcolumn 12 of the microcolumn array, that is, it can be light-transmitting, non-light-transmitting, or semi-light-transmitting. At this time, it is only necessary to change the positions of the optical signal generating device 2 and the optical signal detecting device 3, and arrange them above the base 11. In this way, every time the microcolumn bends or swings, the optical signal received by the optical signal detecting device 3 will change compared with when the microcolumn 12 is upright and not deformed. By analyzing the change of the front and rear optical signals, the relative magnitude of the cellular mechanical force can also be obtained, and the absolute magnitude of the cellular mechanical force can be obtained after calibration with the standard value.

## Example 4: Cellular Mechanical Force Detection Device

As shown in FIGS. 1 and 14, the difference between this example and the first to third examples is that an anti-reflection layer 105 for light is provided on the surface of the microcolumn 12 except for the area where the light reflection layer 13 is provided. Such a design can reduce interference of reflected light signals that may be brought by the surface of the column body, enhance signal-to-noise ratio, and make detection results more accurate. In other specific examples, an anti-reflection layer 105 is provided in the base to further enhance signal-to-noise ratio.

In some examples, the light reflection layer 13 may be a layer of gold foil. In other examples, the light reflection layer 13 may also be other metal layers or other reflective materials with a light reflection function. Different materials may have differences in reflective effect, difficulty in preparation of the reflection layer, cost, etc., and can be considered and selected according to specific conditions in actual operation.

In the first to fourth examples, the cross-sectional shape of the microcolumn 12 is circular. In other embodiments, the cross-sectional shape of the microcolumn 12 may also be elliptical or polygonal. In various specific implementations of the present invention, different cross-sections can achieve different purposes. For example, a circular cross-section has isotropic characteristics, that is, the mechanical properties of the microcolumn itself are not sensitive to direction. While a cross-section such as an ellipse is anisotropic, that is, the mechanical properties of the microcolumn itself are sensitive to direction, which can control the sensitivity to force field in different directions, and can regulate cell tropism to a certain extent (the geometric shape of most cells is actually asymmetric, and cell tropism in the present invention refers to the asymmetry, polarity or directionality exhibited by cells). For example, if the cross-section is elliptical, the cross-section has a major axis and a minor axis, and it is much easier to push the microcolumn along the minor axis than the major axis, so the deformation is greater under relative force conditions. In some extended embodiments, if cells are planted on such microcolumns, the cells and microcolumns have anisotropic mechanical interaction, which will cause cells to grow along one side. And applied to fluid, it can be used to determine the direction of fluid.

In the first to fourth examples, the size of the microcolumn array is: column height 10nm~500μm, column spacing 10nm~50μm, upper surface diameter 50nm~50μm. Microcolumns within this size range can meet the basic use conditions of microcolumns used as sensors, that is, at least deformable and not collapsed. On this basis, regulation of different microcolumn array sizes can also achieve the following functions: for example, regulation of microcolumn aspect ratio (which can be understood as the ratio of height to cross-sectional diameter/side length/major diameter at the microcolumn level) can achieve certain microcolumn deformation performance regulation functions, thereby better simulating the in vivo organ tissue environment (such as bone tissue and nerve tissue with different hardness).

In addition, the overall specification of the array or the number of microcolumns on a certain area of the base 11 will also affect the ligand density, that is, the number of adhesion points that cells can find on the surface. If the microcolumn 12 array is sparser, the number of adhesion points that cells can find is smaller, which will have a significant impact on cell behavior.

The cross-sectional area size in the shape of the microcolumn will also affect cell adhesion behavior, because the formation of focal adhesion by cell adhesion requires a certain area. If it is a nano-microcolumn, the cross-sectional area of the microcolumn is small, which will affect the formation of focal adhesion.

In a word, combined with the material itself characteristics and certain microcolumn array size, better cell support effect, chip stability and measurement accuracy can be achieved. Regulation of the distribution of microcolumn arrays can also regulate and affect cell attachment state to a certain extent.

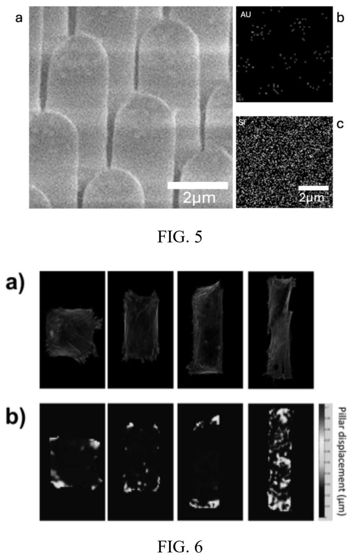

Please refer to FIG. 5, which is a scanning electron microscope image of a microcolumn (polydimethylsiloxane) with a light reflection layer (gold) at the top; wherein, FIG. 5a is a scanning electron microscope image of the microcolumn; FIG. 5b is an element characterization diagram of the top region of the microcolumn; FIG. 5c is an element characterization diagram of the side region of the microcolumn (excluding the top region). By characterizing the material composition of the microcolumn through the scanning electron microscope image in FIG. 5, it can be confirmed that Au element exists at the top position of the microcolumn, and Si element exists at other positions of the microcolumn.

**Example 5: Cellular Mechanical Force Detection Device**

The difference between this example and the first to fourth examples is that the top end face of some microcolumns 12 of the microcolumn array is provided with a substance 106 with cell adhesion effect. This example uses collagen in extracellular matrix molecules. In other embodiments, one or a combination of several of extracellular matrix molecules including collagen, as well as fibronectin, vitronectin, laminin and tropoelastin can also be used. In other embodiments, other types of substances with cell adhesion effect can also be provided on the top end face of all or part of the microcolumns of the microcolumn 12 array, such as extracellular matrix simulate substances, such as polypeptides containing RGD adhesion sequence; or substances 106 with cell adhesion promotion mechanism, including polylysine; or substances interacting with cell surface receptors.

Setting such a substance 106 with cell adhesion effect on the top end face of the microcolumn 12 can effectively promote the adhesion of cells to the microcolumn 12, thereby realizing regulation of cell adhesion, proliferation, migration, state, differentiation, etc. In addition, if a substance with cell adhesion effect is provided on the top end face of some microcolumns in the preset region of the microcolumn array, such as extracellular matrix proteins such as fibronectin, these microcolumns can form a certain shape. Thus, cells tend to adhere to microcolumns at specific positions and shapes, so as to perform high-throughput mechanical measurement under the condition of controlling cell size, shape and tropism characteristics.

The cellular mechanical force detection device of this example, cell adhesion substances are beneficial to the stability of cells/multicellular aggregates on microcolumns.

**Example 6: Cellular Mechanical Force Detection Device**

The difference between this example and the fifth example is that, as described in the fifth example, the top end face of some microcolumns 12 of the microcolumn array is provided with a substance 106 with cell adhesion effect, while in this example, the column surface (end face or side surface) of the part of microcolumns 12 whose top end face is not provided with a substance with cell adhesion effect is also provided with a substance with cell adhesion inhibition effect, such as F-127. In other specific examples, the base is provided with a substance 107 with cell adhesion inhibition effect. The setting of the substance 107 with cell adhesion inhibition effect makes cells more inclined to adhere to microcolumns at specific positions and shapes (on the top), so as to perform high-throughput mechanical measurement under the condition of controlling cell size, shape and tropism characteristics, making cellular mechanical force act on microcolumns through the top of microcolumns.

**Example 7: Cellular Mechanical Force Detection Device**

The difference between this example and the first to fourth examples is that the top end face of the microcolumn 12 array provided with a substance with cell adhesion effect forms a preset pattern. Specifically, a microcontact printing technology can be used to print a specific pattern of cell adhesion molecule layer to promote cell adhesion in these regions. The so-called preset pattern can be triangle, quadrilateral, polygon, circle, ellipse and other shapes. The functions of the preset pattern include: first, controlling cell-cell contact through these patterns composed of substances with cell adhesion effect, so as to facilitate the demand for high-throughput data acquisition. Second, the cell shape can be unified to achieve dimensionality reduction in data processing, thereby reducing analysis difficulty. Third, limiting cell adhesion area to control cell size, shape, tropism, differentiation state, etc., and even regulating cell mechanical state through controlling actin filaments, so as to meet the requirements of some special technical requirement scenarios.

In another example roughly similar to this example, the part not printed with the preset pattern can be treated with a substance with cell adhesion inhibition effect such as BSA (bovine serum albumin) or F127 (polymeric nonionic surfactant) to inhibit cell adhesion in these regions, thereby realizing directional adhesion, control of cell morphology or simulation of specific cell microenvironment.

In other examples, fibronectin (FN) is used as an exemplary description of the substance with cell adhesion effect, but not intended to limit the implementation of the present invention. Microstamps with protruding square and rectangular patterns on the surface are used respectively, and fibronectin is adhered to the surface of the microstamp. The fibronectin on the protruding part of the stamp is transferred to the top of the metal reflection layer on the top of the microcolumn by microcontact printing. Then, the microcolumn is immersed in F-127 solution, so that the part not provided with fibronectin has the effect of inhibiting cell adhesion. Finally, after the microcolumn is thoroughly cleaned with physiological saline, fibroblasts with cell membrane staining are planted on the surface of the microcolumn, and then fluorescence imaging is performed on the cells (as shown in a in FIG. 6), and high-resolution measurement of the force field in the cells is performed at the same time (as shown in b in FIG. 6). Please refer to a and b in FIG. 6. a in FIG. 6 is a fluorescence imaging diagram of cells adhering to a preset pattern composed of microcolumn groups with fibronectin on the top, and it can be seen that the adhesion range of cells is limited to the region with fibronectin; based on this, cell adhesion area can be limited by preset patterns, and then mechanical monitoring of cells under the condition of controlling cell size, shape, tropism, differentiation state, etc. b in FIG. 6 is a cellular mechanical force distribution diagram calculated from light reflection signals measured on microcolumns.

In other examples, OKT3 antibody (i.e., a substance interacting with cell surface receptors) or fibronectin (FN) is used as an exemplary description of the substance with cell adhesion effect, but not intended to limit the implementation of the present invention. Please refer to a-d in FIG. 7. a in FIG. 7 is a schematic diagram of an experiment using OKT3 antibody as a substance with cell adhesion effect; the upper images in b in FIG. 7 are fluorescence imaging diagrams of cells adhering to the top of microcolumns with OKT3 antibody and fibronectin on the top respectively; the lower images in b in FIG. 7 are distribution diagrams of light reflection signals (reflecting cellular mechanical force magnitude) measured on microcolumns; c in FIG. 7 is a comparison diagram of mechanical force magnitude measured on surfaces coated with OKT3 antibody and fibronectin; d in FIG. 7 is a diagram of dynamic changes in cellular mechanical force of T cells after being planted on the surface (microcolumn top) coated with OKT3 antibody. Specifically, in this experiment, OKT3 antibody or fibronectin can be coated on the top of part of the microcolumns of the same or different cellular force detection devices, and T cells are planted on the surface of the cellular mechanical force detection device with cell adhesion substances. It can be known from a-d in FIG. 7 that the cellular mechanical force detection device with a substance (such as OKT3 antibody) interacting with cell surface receptors or fibronectin (FN) coated on the surface can be used to real-time monitor the influence of the substance on cellular mechanical force and interaction.

**Example 8: Cellular Mechanical Force Detection Device**

The difference between this example and the first to seventh examples is that the cellular mechanical force detection device further includes a cell restriction mechanism, and the cell restriction mechanism includes one or several restriction surfaces 16. The restriction surface 16 is a plane or curved surface perpendicular to the plane where the base 11 is located, connected to the base 11 or integrally formed with the base 11, and the height of the restriction surface 16 is higher than the microcolumns 12 and surrounds a preset number of microcolumns 12.

The function of the cell restriction mechanism in this example is single-cell isolation detection, that is, to avoid contact or adhesion between cells during detection, and to limit cell morphology, so as to facilitate high-throughput testing. According to different requirements, the number of restriction surfaces 16 in the cell position restriction mechanism or the enclosed shape can be different. For example, the cell position restriction mechanism may include a cylindrical surface, or 3 planes connected end to end to form a triangular cross-sectional shape and enclose a certain number of microcolumns, 4 planes perpendicular to each other and connected end to end to form a rectangular shape to enclose a certain number of microcolumns, N planes connected end to end to enclose an N-gon, or a curved surface with a circular cross-section, etc. That is to say, the cross-sectional shape formed by the restriction surface 16 is a controllable closed shape, and its area (or the number of microcolumns that can be accommodated in its space) is also controllable.

In actual implementation, according to different manufacturing processes, the cell restriction mechanism can also appear in the following forms:

A. Please refer to FIG. 8, which is a schematic structural diagram a of a cellular mechanical force detection device with a cell restriction mechanism. In the figure, the cell restriction mechanism is integrally formed with the base 11, that is,: the substance forming the cell restriction mechanism has several recessed spaces 15, the wall surface of the recessed space 15 is the restriction surface 16, the depth of the recessed space 15 is the height of the restriction surface 16, the bottom of the recessed space 15 is the base 11, and each recessed space 15 has several microcolumns 12.

B. Please refer to FIG. 9, which is a schematic structural diagram b of a cellular mechanical force detection device with a cell restriction mechanism. In the figure, the restriction surface 16 is a structure bonded to the base 11.

## Example 9: Cellular Mechanical Force Detection Device

The difference between this example and the eighth example is that the cell restriction mechanism in this example is a silicon film.

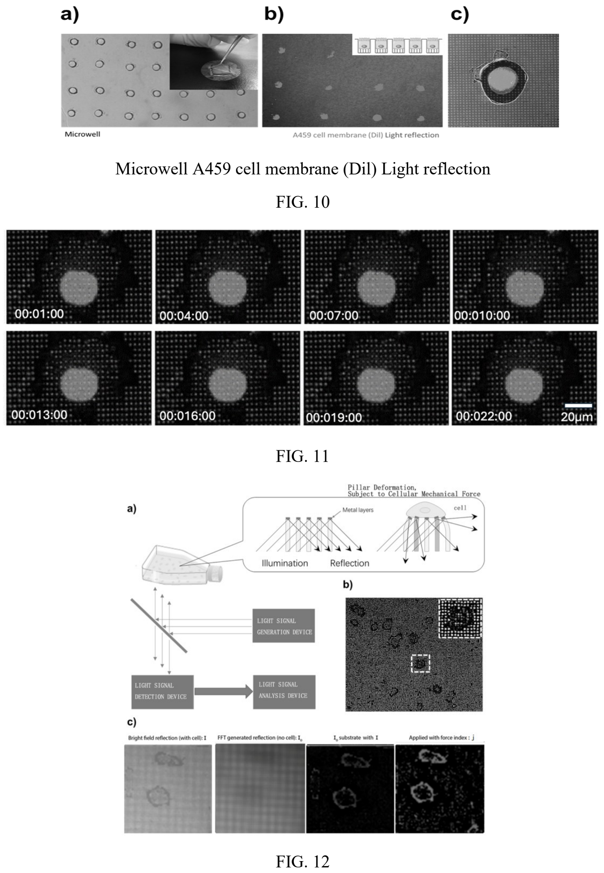

Specifically, please refer to a-c in FIG. 10. a in FIG. 10 is a physical picture of a cellular mechanical force detection device using a silicon film as a cell restriction mechanism. In a in FIG. 10, after laser drilling, the silicon film is adhered to the base, and each hole is provided with microcolumns. The silicon film limits cell morphology and migration, and controls contact or adhesion between cells; b in FIG. 10 is a fluorescence microscope picture of the cellular mechanical force detection device using a silicon film as a cell restriction mechanism under light reflection; c in FIG. 10 is an enlarged view of b in FIG. 10. In some examples, the size of each hole of the silicon film can be set to match the size of a single cell, suitable for single-cell adhesion, thereby limiting cell contact, cell morphology and migration range.

## Example 10: Cell and/or Multicellular Aggregate Characterization System

A characterization system for cell/multicellular aggregate interaction, including the cellular mechanical force detection device 1, light signal generation device 2 and light signal detecting device 3 described in the first or second example; the light signal generation device 2 and light signal detecting device 3 are both located below the base 11 in the cellular mechanical force detection device 1, the light signal generation device 2 has a light source, and the light emitted by the light source irradiates the light reflection layer 13 of the microcolumn 12 through the incident light path (successively passing through the light-transmitting base and the light-transmitting microcolumn body), is reflected, and the reflected light enters the light signal detecting device 3 through the reflected light path (successively passing through the light-transmitting microcolumn body and the light-transmitting base). The light signal detecting device 3 can acquire reflected light signals before and after the microcolumn 12 contacts with cells. In other examples, such a characterization system for cell/multicellular aggregate interaction further includes a light signal analysis device 4, which can obtain cellular mechanical force information including the magnitude, direction, and changes within a certain time range of cellular mechanical force by comparing, analyzing, and calculating the reflected light signals before and after the microcolumn 12 contacts with cells.

## Example 11: Cell and/or Multicellular Aggregate Characterization System

Please refer to FIG. 4, which is a characterization system for cell/multicellular aggregate interaction related to the eleventh example of the present invention, including the cellular mechanical force detection device 1 described in the third example, and further including a light signal generation device 2 and a light signal detecting device 3; the light signal generation device 2 and the light signal detecting device 3 are both located above the base 11 in the cellular mechanical force detection device 1, the light signal generation device 2 has a light source, and the light emitted by the light source irradiates the light reflection layer 13 through the incident light path, is reflected, and the light signal detecting device 3 can acquire reflected light signals before and after the microcolumn 12 contacts with cells.

In the examples of the present invention, the light signal detecting device can be a microscope, charge-coupled device CCD, complementary metal oxide semiconductor CMOS, photomultiplier tube PMT and photoelectric converter PT, film, or other light signal detection elements with the same function, which is not specifically limited in the present invention. It should be noted that in some examples of the present invention, when a microscope is used as the light signal detecting device, there is no need to set an independent light signal generation device, and the cellular mechanical force detection device of the present invention can be directly placed on the stage of the microscope, using the light source of the microscope as the light signal generation device and the objective lens 101 of the microscope (5x objective lens is sufficient, no need to rely on high-magnification optical objective lens) as the light signal detecting device; when other light signal detecting devices such as charge-coupled device CCD are used, an independent light signal generation device needs to be set. In the examples of the present invention, the light signal generation device can be LED, halogen lamp, laser (for example, infrared laser), or other light sources, or other devices with these light sources, which is not specifically limited in the present invention.

The following specifically introduces the visualization process of monitoring cellular mechanical force by the characterization system for cell/multicellular aggregate interaction related to this example.

Please refer to FIG. 11, which is a fluorescence microscope image of monitoring cellular mechanical force by the characterization system for cell/multicellular aggregate interaction of this example. Specifically, cells (for example, fibroblasts Fibroblast in this example) are placed on the cellular mechanical force detection device, and the light signal detecting device (for example, microscope) can convert cellular mechanical force information into optical signals and form images for visualization observation, and can feedback changes in cellular mechanical force in real-time.

**Example 12: Cell and/or Multicellular Aggregate Characterization System**

Please refer to FIG. 4, which is a characterization system for cell/multicellular aggregate interaction related to the twelfth example of the present invention, including the cellular mechanical force detection device 1 described in the third example, and further including a light signal generation device 2, a light signal detecting device 3 and a light signal analysis device 4. The light signal generation device 2 and the light signal detecting device 3 are both located above the base 11 in the cellular mechanical force detection device 1; the light signal generation device 2 has a light source, and the light emitted by the light source irradiates the light reflection layer 13 through the incident light path, is reflected; the beam splitter 5 can be a half-transparent half-reflective or other equivalent optical element, and its main purpose is to simplify the design of the light path; the light signal detecting device 3 can acquire reflected light signals before and after the microcolumn 12 contacts with cells; the light signal analysis device 4 can obtain cellular mechanical force information including the magnitude, direction, and changes within a certain time range of cellular mechanical force by comparing, analyzing, and calculating the reflected light signals before and after the microcolumn 12 contacts with cells.

In the examples of the present invention, the light signal analysis device can be optical image analysis software ImageJ, Matlab, Fluoview, Python, or other optical image analysis elements with the same function, or combined use of these analysis software, which is not specifically limited in the present invention.

The following specifically introduces the detection and analysis process of the characterization system for cell/multicellular aggregate interaction related to this example.

Please refer to a-c in FIG. 12. a in FIG. 12 is a schematic structural diagram of a characterization system for cell/multicellular aggregate interaction; b in FIG. 12 is an image of light reflection signals of the cellular mechanical force detection device acquired by the light signal detecting device; c in FIG. 12 is a visualization effect diagram of mechanical force magnitude and distribution.

As shown in a in FIG. 12, on the cellular mechanical force detection device, the top of each microcolumn is provided with a metal reflection layer, and the side is provided with an anti-reflection layer; when there is no cell, light irradiates the microcolumn from below, which will be completely reflected and fully received by the light signal detecting device (such as CCD camera); but when cells adhere to the microcolumn, the cellular force generated by cell movement will cause the microcolumn to tilt, thereby reducing the reflection signal. After analysis of the light reflection signal, the cell force intensity can be calculated.

Further, the light signal detecting device (such as CCD camera) is used to collect images of light reflection signals of the cellular mechanical force detection device and enlarged images of local cell adhesion regions (as shown in b in FIG. 12). Then, the light signal analysis device is used to further process the image in b in FIG. 12 to convert it into a more intuitive visualization effect diagram of mechanical force magnitude and distribution 12c.

The specific processing process is as follows: first, based on b in FIG. 12, obtain a bright field reflection signal image (I, focused on the cell); then, filter high-frequency signals after Fourier transform of the image, and perform inverse Fourier transform operation to calculate and obtain a microcolumn reflection signal image (I0) without offset; then, further process I and I0 images to convert the reflection signal image into a more intuitive cellular mechanical force image (I0 signal value minus I signal value), and then standardize to obtain a more intuitive cellular mechanical force intensity diagram j.

Examples 1 to 9 of the present invention can also measure hardness at the same time, and the hardness of cells and/or multicellular aggregates can be measured by rotating the microcolumn into the cell under the action of external force.

**Example 13: Method for Calculating Mechanical Force, Relationship Between Mechanical Force and Light Reflection Signal**

This example combines the characterization system for cell/multicellular aggregate interaction described in any one of Examples 10 to 12 or the cellular mechanical force detection method described in Example 14 to illustrate the method for calculating mechanical force in the examples of the present invention; and uses fluid as an external force to verify the relationship between mechanical force and light reflection signal.

Please refer to FIG. 13, where a in FIG. 13 is a schematic structural diagram of the cellular mechanical force detection device in a microfluidic environment before and after fluid is turned on; b and c in FIG. 13 are comparison diagrams of bright field microscope images, reflected light signal distribution diagrams and their superimposed effect diagrams of microcolumns before and after fluid is turned on; d in FIG. 13 is a light reflection signal intensity diagram before and after fluid is turned on; e in FIG. 13 is a linear relationship diagram and linear range between light reflection signal and microcolumn offset. Wherein, the superimposed effect diagram refers to a diagram formed by superimposing the bright field microscope image of microcolumns and the reflected light signal distribution diagram.

First, as shown in FIG. 13, the cellular mechanical force detection device is integrated into the microfluidic channel. When the applied flow rate increases, the microcolumn generates displacement and changes the angle of the reflection layer on the microcolumn surface (a-c in FIG. 13). It can be seen from d in FIG. 13 that before and after the fluid is turned on, the light reflection signal changes from strong to weak. Specifically, the offset of the microcolumn is changed by the strength of the flow rate, and the displacement of the top of the microcolumn relative to the bottom of the microcolumn is photographed by a confocal microscope. The mechanical force on each microcolumn can be calculated by the following formula:

$$F = k_{\text{bend}}\delta = \frac{3}{64}\pi E \frac{D^4}{L^3}\delta$$

Wherein, F represents the mechanical force causing the microcolumn to deflect by $\delta$ angle, E represents Young's modulus, kbend represents the ideal spring constant of the isolated nanocolumn, D represents the diameter of the microcolumn, and L represents the height of the microcolumn.

At the same time, record the light reflection signal on the top of the microcolumn, and plot e in FIG. 13 between the light reflection signal and the microcolumn offset to obtain a linear relationship diagram and linear range between the light reflection signal (reflecting cellular mechanical force) and the microcolumn offset.

**Example 14: Method for Detecting Cellular Mechanical Force**

A method for detecting cellular mechanical force, comprising the following steps:
Using the light signal generation device 2 in the characterization system for cell/multicellular aggregate interaction described in any one of Examples 10 to 12 to emit light.

Using the light signal detecting device 3 in the characterization system for cell/multicellular aggregate interaction described in any one of Examples 10 to 12 to detect the light acted by the cellular mechanical force detection device 1. The light signal detecting device 3 can acquire reflected light signals before and after the microcolumn 12 in the cellular mechanical force detection device 1 contacts with cells. In other embodiments, the light signal analysis device 4 in the characterization system for cell/multicellular aggregate interaction obtains cellular mechanical force information including the magnitude, direction, and changes within a certain time range of cellular mechanical force by comparing, analyzing, and calculating the reflected light signals before and after the microcolumn 12 contacts with cells.

Please refer to a-d in FIG. 14. a and b in FIG. 14 are schematic structural diagrams of microcolumns of the cellular mechanical force detection device before and after contact with cells; b in FIG. 14 is a reflected light signal acquired by the light signal detecting device (CCD electronic photosensitive element), and obvious reflection signal attenuation can be seen in the region with large force field around the cell; c in FIG. 14 is a monitoring diagram of cell migration process (after staining the cell membrane, excited with a fluorescent light source, and recorded by the CCD electronic photosensitive element); d in FIG. 14 is a reflected light signal distribution diagram during cell migration process (recorded by the CCD electronic photosensitive element). It can be seen from c and d in FIG. 14 that the reflection signal is significantly attenuated in the part where the cell exerts force. d in FIG. 14 shows the reflected light signal monitored in real-time during cell migration, and the mechanical force during migration is fed back in real-time through the reflected light signal. It can be seen that monitoring the reflected light signal during cell migration through the light signal detecting device can feed back changes in cellular mechanical force during cell migration in real-time.

**Example 15: Method for Preparing a Cellular Mechanical Force Detection Device**

A method for preparing a cellular mechanical force detection device, comprising the following steps: laying a layer of light reflection layer 13 on the top or upper half column surface of the microcolumn 12 to obtain a microcolumn 12 with a reflection layer on the top or upper half column surface.

**Example 16: Method for Preparing a Cellular Mechanical Force Detection Device**

A method for preparing a cellular mechanical force detection device, comprising the following steps: Uniformly plating a layer of anti-reflection layer on the entire microcolumn 12; removing the anti-reflection layer on the top or upper half column surface; laying a layer of light reflection layer 13 on the top or upper half column surface of the microcolumn 12.

**Example 17: Method for Preparing a Cellular Mechanical Force Detection Device**

The difference between this example and Examples 15 and 16 is that the step of "laying a layer of light reflection layer 13 on the top or upper half column surface of the microcolumn 12" specifically refers to: uniformly sputtering a layer of reflective metal on the top or upper half column surface of the microcolumn to obtain a microcolumn with a metal light reflection layer on the top or upper half column surface.

**Example 18: Method for Cell Identification (Including Visual Qualitative Identification and Accurate Qualitative and Quantitative Identification)**

This example specifically provides use of cellular mechanical force information acquired by the characterization system for cell/multicellular aggregate interaction described in any one of Examples 10 to 12 or the cellular mechanical force detection method described in Example 14 in a method for identifying cells.

Please refer to a to g in FIG. 15. a in FIG. 15 is a fluorescence imaging diagram of a mixed system of healthy cells and lung non-small cell cancer cells; b in FIG. 15 is a distribution diagram of light reflection signals of the cellular mechanical force detection device acquired by the light signal detecting device; c in FIG. 15 is a visualization effect diagram of mechanical force magnitude and distribution; d in FIG. 15 is an enlarged view of representative single-cell force distribution of healthy cells and lung non-small cell cancer cells in c in FIG. 15; e in FIG. 15 is a comparison diagram of cell morphology between healthy cells and lung non-small cell cancer cells; f in FIG. 15 is a comparison diagram of reflected signal intensity between healthy cells, lung non-small cell cancer cells, and a mixture of these two cells in different proportions; g in FIG. 15 is a cluster analysis diagram obtained by structuring c in FIG. 15 and processing based on structured cellular information.

Specifically, in this example, healthy cells (Normal) and lung non-small cell cancer cell lines (Cancer) are used as detection objects, and cell membranes of healthy cells and cancer cells are pre-stained with two different fluorescent dyes (Dil & DIO), mixed in a certain proportion, and added to the same cellular mechanical force detection device (in other embodiments, they can be added to different independent cellular mechanical force detection devices).

Further, the light signal detecting device (microscope is used in this example) is used to collect images of light reflection signals of the cellular mechanical force detection device (as shown in b in FIG. 15), and high-resolution force field distributions in the two types of cells are directly rendered by the light signal detecting device to be converted into readable light intensity attenuation signals (reflecting cell force intensity) and displayed in the picture (as shown in c in FIG. 15). According to the difference in light attenuation degree between the two types of cells shown in the picture c in FIG. 15, the two types of cells can be visually distinguished by naked eyes (qualitative analysis).

Further, the light signal analysis device is used to further process the light reflection signal in c in FIG. 15. Specifically, in this example, the light signal analysis device (ImageJ and Python analysis software are used in this example, and other image analysis software can also be used in other examples) is used to collect information from the obtained cell force field diagram 15c, which includes collecting cellular mechanical force magnitude information at multiple points of each cell, thereby obtaining multiple cellular mechanical force magnitude data at multiple points in multiple cells; preprocessing the acquired cellular mechanical force magnitude information to form structured cellular information; and analyzing to obtain a comparison result diagram of cell morphology between healthy cells and lung non-small cell cancer cells (as shown in e in FIG. 15).

The structured cellular information includes the number of cells, the number of cell features, and feature information of each cell feature (such as cell adhesion area and cell circularity in this example). At this time, the structured cellular information can be regarded as a two-dimensional matrix of a feature matrix, where N is the number of cells, P is the number of cell features, and P=2 here, that is, the cell features are: cellular mechanical force magnitude, distribution of cellular mechanical force in the cell.

Further, the above structured cellular information is used as input data to establish a cell feature model using supervised machine learning (compared with two cell lines pre-stained with different cell membrane dyes (Dil & DIO)), and a large amount of structured cellular information of cells is used to train the cell feature model to obtain a cluster analysis diagram as shown in g in FIG. 15, and then the obtained cell feature model is applied to classification and identification of unknown types or unknown states of cells. It can be seen that according to the structured cell feature data (cellular mechanical force magnitude, distribution of cellular mechanical force in the cell) as input data, normal healthy cells and cancer cells can be clustered and typed by the light signal analysis device (ImageJ and Python analysis software are used in this example, and other cluster analysis software can also be used in other examples), thereby realizing identification of unknown cell types.

e in FIG. 15 shows that there is no statistically significant difference in morphology (including cell adhesion area and cell circularity) between different cells; f in FIG. 15 shows obvious differences in reflection signal intensity (reflecting cell force) between normal cells and tumor cells, and the reflection signal intensity has a certain linear relationship with the mixing ratio when normal cells and tumor cells are mixed in a certain proportion. It can be seen that compared with other features of cells (such as cell adhesion area and cell circularity in e in FIG. 15), the cellular mechanical features measured by the cellular mechanical force detection device of the present invention can more intuitively and accurately identify the state and type of cells (quantitative and qualitative analysis), and it is confirmed that using the cell force field as a marker can better distinguish cell types.

In conclusion, based on the cellular mechanical force detection device of the present invention, not only qualitative analysis can be performed through visual distinction by naked eyes, but also more intuitive and accurate identification (quantitative and qualitative analysis) of the state and type of cells can be performed based on the measured cellular mechanical features, and it is confirmed that using the cell force field as a marker can better distinguish cell types.

The above cell identification method is applicable to typing and identification of cells/multicellular aggregates before, during and after the action of various factors including external factors described in Implementation Mode 1 on cells/multicellular aggregates.

**Example 19: Method for Detecting Cell Viability**

This example specifically provides use of cellular physical information acquired by the characterization system for cell/multicellular aggregate interaction described in any one of Examples 10 to 12 or the cellular mechanical force detection method described in Example 14 in monitoring cell viability.

Please refer to a-c in FIG. 16. a in FIG. 16 is a schematic diagram of the operation process of the cell viability detection method; b in FIG. 16 is a comparison diagram of cell viability measured by the MTT method and cellular mechanical force measured by the device or system or method of the present invention after A549 cells are treated with different doses of 5FU for 24h; c in FIG. 16 is a comparison diagram of cell viability measured by MTT method and cellular mechanical force measured by the device or system or method of the present invention after A549 cells are treated with different doses of 5FU for different times.

Specifically, in this example, non-small cell lung cancer cells A549 are cultured on multiple cellular mechanical force detection devices, treated with different doses of the cell proliferation inhibitory drug 5-fluorouracil (5-FU), and cellular mechanical force at different time points is monitored by the characterization system for cell/multicellular aggregate interaction described in any one of Examples 10 to 12 or the cellular mechanical force detection method described in Example 14. Cell proliferation and cytotoxicity at different time points are monitored by CCK-8 kit, and cell viability measured by MTT assay is used as a control group to obtain data in FIGS. 16b and 16c.

It can be seen from b and c in FIG. 16 that after measurement by the traditional MTT assay and the device or system or method of the present invention, both the cell viability measured by the MTT assay and the cell viability reflected by the cellular mechanical force show a gradually decreasing trend in a dose-dependent manner, that is, cellular mechanical force is positively correlated with cell viability.

In addition, as shown in b in FIG. 16, after treatment with different doses of 5FU for 24h, compared with the control group DMSO, the reduction range of cell viability reflected by mechanical force is larger than that reflected by the traditional MTT assay, thereby more intuitively evaluating cell viability. As shown in c in FIG. 16, within 12h of 5FU treatment, the change in cell viability measured by the MTT method is not obvious; while by measuring mechanical force, a decrease in cellular mechanical force can be observed at an earlier time point before the decrease in cell metabolic activity is detected by the MTT method. Specifically, a significant decreasing trend can be observed at 6h under the treatment dose of 0.5 $\mu$M, and a significant decreasing trend can be observed at 3h under the treatment dose of 1 $\mu$M, thereby more sensitively

characterizing the decrease in cell viability.

In conclusion, this example shows that directly detecting cellular mechanical force through the cellular mechanical force detection device is a highly sensitive and effective method for evaluating cell viability in response to drugs.

**Example 20: Characterization Method Based on Interaction Between Cells and/or Multicellular Aggregates**

This example provides acquiring cellular physical information of cells/multicellular aggregates through the cellular mechanical force detection device or characterization system described in any one of the above examples or the cellular mechanical force detection method described in any one of the above schemes; specifically, it includes the following steps:

Placing cells/multicellular aggregates on the cellular mechanical force detection device, adding cell culture medium to at least make the cells/multicellular aggregates contact with the cell culture medium, standing for more than half an hour, continuing to add cell culture medium for culture until the cells/multicellular aggregates are completely attached to the microcolumns, and then measuring and acquiring cellular mechanical force of the cells/multicellular aggregates.

In some specific examples, external stimuli are added to detect dynamic changes in cellular mechanical force of cells/multicellular aggregates under external stimuli;

Wherein, external stimuli can be added before or during detection of cellular mechanical force.

Different cells/multicellular aggregates can be divided into: two or more different cells/multicellular aggregates, one cell/multicellular aggregate in different states formed at different times or under external stimuli, two or more different regions in one cell/multicellular aggregate;

Two or more different cells/multicellular aggregates: can be pathological cells/multicellular aggregates and normal cells/multicellular aggregates.

**Example 21: Typing and Identification Method Based on Interaction Between Cells and/or Multicellular Aggregates**

This example provides acquiring cellular physical information of cells/multicellular aggregates through the cellular mechanical force detection device or characterization system described in any one of the above examples or the cellular mechanical force detection method described in any one of the above schemes; identifying cells/multicellular aggregates before, during and after the interaction between cells and/or multicellular aggregates according to the cellular physical information;

Specifically including:

S1. Acquiring visualized cellular information including cells/multicellular aggregates before, during and after the interaction between cells/multicellular aggregates through the cellular mechanical force detection device, including visualized information such as changes in cellular mechanical force magnitude and distribution;

S2. Using the visualized information for visualized identification of to be measured cells and aggregates.

In some specific examples,

The cellular mechanical force detection device further includes a cell restriction mechanism, and the cell restriction mechanism includes one or several restriction surfaces, which are planes or curved surfaces perpendicular to the plane where the base is located, connected to the base or integrally formed with the base, and the height of the restriction surfaces is higher than the microcolumns and surrounds a preset number of microcolumns. The first interacting cells/multicellular aggregates and the second cells/multicellular aggregates can be restricted in specific regions to facilitate observation of changes in their respective cellular mechanical force, and the first cells/multicellular aggregates and the second cells/multicellular aggregates have the opportunity to contact, promoting their interaction, and the contact area is fixed, facilitating the first cells/multicellular aggregates and the second cells/multicellular aggregates to remain as two independent cell groups during interaction, facilitating observation and subsequent data analysis and quantification.

Optionally, the cell restriction mechanism is a silicon film, and several holes are arranged in the silicon film, microcolumns are arranged in the holes, and each hole accommodates more than one cell or more than one multicellular aggregate. A fixed number of cell groups can be cultured in the microwells in an orderly manner.

**Example 22: Method for Identifying Cells and/or Multicellular Aggregates**

This example provides acquiring cellular physical information of cells and/or multicellular aggregates through the cellular mechanical force detection device or characterization system described in any one of the above examples or the cellular mechanical force detection method described in any one of the above schemes, and typing and identifying cells and/or multicellular aggregates according to the cellular physical information;

The cellular physical information is acquired under at least one of the following conditions:

(1) an interaction between cells and multicellular aggregates;

(2) an interaction between cells;

(3) an interaction between multicellular aggregates;

(4) cells and/or multicellular aggregates at different growth times;

(5) different regions within multicellular aggregates;

(6) an effect of substances on cells and/or multicellular aggregates;

(7) an effect of other physical, biological or chemical factors on cells and/or multicellular aggregates;

Specifically including:

S1. Adhering cells/multicellular aggregates to microcolumns, and measuring cellular physical information;

S2. During or after adding other cells/multicellular aggregates, substances or applying other actions, acquiring cellular information of the adhered cells/multicellular aggregates, where the cellular information includes cellular physical information at a certain point in the cells/multicellular aggregates acquired based on the cellular mechanical force detection device, and the cellular physical information includes the magnitude of cellular mechanical force at that point, specifically: using the light signal detecting device (or combined with the light signal analysis device) to collect cellular information of multiple cells on the cellular mechanical force detection device, which includes collecting cellular mechanical force magnitude information at multiple points of each cell, thereby acquiring multiple cellular mechanical force magnitude data at multiple points in multiple cells;

S3. Preprocessing the acquired cellular information to form structured cellular information; the structured cellular information includes the number of cells, the number of cell features, and feature information of each cell feature. At this time, the structured cellular information can be regarded as a two-dimensional feature matrix, where N is the number of cells, P is the number of cell features, and P=1 here, that is, the cell feature is cellular mechanical force magnitude;

S4. Using the structured cellular information as input data to establish a cell feature model using supervised, unsupervised or semi-supervised machine learning, and applying the cell feature model to typing and clustering of cells and/or multicellular aggregates at different growth times and changing states under other factors, and further identifying different types.

In other examples, the cellular physical information further includes the direction of cellular mechanical force at that point. In other examples, the cellular physical information further includes changes in the magnitude or direction of cellular mechanical force at that point within a certain time interval. In other examples, the cellular information further includes cell morphology information. In other examples, cellular physical information is acquired by the method of Example 20. In other examples, the cellular mechanical force detection device further includes a cell restriction mechanism, which keeps the first interacting cells/multicellular aggregates and the second cells/multicellular aggregates independent but able to interact with each other.

The cellular mechanical force detection device of the present invention can not only perform qualitative analysis through visual distinction by naked eyes, but also more intuitively and accurately identify the state of cells/multicellular aggregates based on the measured cellular mechanical features (quantitative and qualitative analysis), and it is confirmed that using the cell force field as a marker can better distinguish types.

Example 23: Method for Evaluating Interaction Between Cells and Cell Aggregates Based on Cellular Mechanical Force

This example provides acquiring cellular physical information of cells and/or multicellular aggregates through the cellular mechanical force detection device or characterization system described in any one of the above examples or the cellular mechanical force detection method described in any one of the above schemes, and typing and identifying cells and their aggregates under interaction according to the cellular physical information. The method is:

S1. Using polydimethylsiloxane (PDMS) material to make a film with a thickness of 5-10 microns through microfabrication technology, and creating multiple microwells with a diameter of about 200 microns on the film. These PDMS microwell films are treated with Pluronic F127 solution and dried, and then accurately placed on the microcolumn array of the cellular mechanical force detection device.

S2. Uniformly distributing lung cancer tumor cells on the above device, so that each microwell with a diameter of 200 microns can accommodate about 20 tumor cells, thereby forming cell aggregates. After one day of culture, unattached cells are washed away. At this time, record the cellular mechanical force signal of the cell aggregates in each microwell.

S3. Adding monocyte cells THP-1, and starting real-time monitoring of changes in cellular mechanical force of cell aggregates. This process not only promotes orderly culture of fixed number of cell groups in microwells, but also facilitates high-throughput monitoring of changes in their mechanical force. The design of the device of the present invention ensures that each cell aggregate has the opportunity to contact with another type of cell, and since the growth of cell aggregates in microwells is restricted, their contact area with the chip is fixed, which is greatly beneficial to subsequent data analysis and quantification.

In addition, the use of this microwell device is not limited to tumor cells, but also applicable to research on cell aggregates (spheroids) or organoids, and can effectively observe the influence of various cells on cell aggregates or organoids. This method provides an efficient and accurate experimental platform for research on cellular mechanical force.

**Example 24: Method for Evaluating Effect of Immune Cells on Tumor Multicellular Spheroids Through Cellular Mechanical Force**

This example provides acquiring cellular physical information of cells and/or multicellular aggregates through the cellular mechanical force detection device or characterization system described in any one of the above examples or the cellular mechanical force detection method described in any one of the above schemes, and further identifying the interaction between immune cells and tumor multicellular spheroids. The specific operation steps are as follows:

S1. Using polydimethylsiloxane (PDMS) material to prepare a microwell structure film with a thickness of 5-10 microns and each microwell with a diameter of about 200 microns through microfabrication technology. The PDMS microwell film is soaked in Pluronic F127 solution, dried, and then laid on the microcolumn array of the cellular mechanical force detection device.
Subsequently, lung cancer tumor cells A549 are uniformly distributed on the above device, and each microwell with a diameter of 200 microns accommodates about 20 tumor cells to form cell aggregates. After one day of culture, unattached cells are removed, and the cellular mechanical force signal of the cell aggregates in each microwell is recorded.

S2. Adding T cell Jurkat cells to the system, and starting real-time monitoring of changes in cellular mechanical force of tumor cell aggregates. This method not only enables orderly culture of fixed number of cell groups in microwells, but also helps high-throughput monitoring of changes in their cellular mechanical force. Through the device of the present invention, each cell aggregate is ensured to have the opportunity to contact with immune cells, thereby effectively monitoring the response of tumor cells to immune cells.

This example, through carefully designed experimental steps, can not only orderly culture fixed number of tumor cell aggregates in the microwell structure, but also evaluate the effect of immune cells on tumor multicellular spheroids at the cellular level. By real-time monitoring changes in cellular mechanical force, this example provides a novel and effective method for evaluating the killing effect of immune cells on tumor cells.

**Example 25: Method for Evaluating Effect of Tumor Cells on Mesothelial Cells Through Cellular Mechanical Force**

This example provides acquiring cellular physical information of cells and/or multicellular aggregates through the cellular mechanical force detection device or characterization system described in any one of the above examples or the cellular mechanical force detection method described in any one of the above schemes, which is acquired based on the effect of tumor cells on vascular endothelial cells, thereby realizing characterization, typing identification and evaluation. The specific steps are as follows:

S1. Preparing a structural film containing microwells with a diameter of about 200 microns using polydimethylsiloxane (PDMS) material with a thickness of 5-10 microns. The PDMS microwell film is soaked in Pluronic F127 solution, dried, and then laid on the microcolumn array of the cellular mechanical force detection device.

S2. Uniformly distributing mesothelial cell line Met5A on the cellular mechanical force detection device, and each microwell with a diameter of 200 microns accommodates about 20 endothelial cells to form cell aggregates. After one day of culture, unattached cells are removed, and the cellular mechanical force signal of mesothelial cell aggregates in each microwell is recorded.

S3. Adding ovarian cancer cell line OVCAR3 to the system, and starting real-time monitoring of changes in cellular mechanical force of mesothelial cells. This example not only orderly cultures fixed number of mesothelial cells in microwells, but also can identify the interaction between tumor cells and mesothelial cells by high-throughput monitoring of changes in cellular mechanical force.

Through this example, the influence of tumor cells on mesothelial cells can be effectively monitored and analyzed, providing a new experimental method and technical means for studying the angiogenesis process of tumors and their mechanism of action on mesothelial cells.

Example 26: **Method for Evaluating Effect of Tumor Cells on Vascular Endothelial Cells Through Cellular Mechanical Force**

This example provides acquiring cellular physical information of cells and/or multicellular aggregates through the cellular mechanical force detection device or characterization system described in any one of the above examples or the cellular mechanical force detection method described in any one of the above schemes, which is acquired based on the effect of tumor cells on vascular endothelial cells, thereby realizing characterization, typing identification and evaluation. The specific steps are as follows:

S1. Preparing a structural film containing microwells with a diameter of about 200 microns using polydimethylsiloxane (PDMS) material with a thickness of 5-10 microns. The PDMS microwell film is soaked in Pluronic F127 solution, dried, and then laid on the microcolumn array of the cellular mechanical force detection device.

S2. Uniformly distributing vascular endothelial cell line HUVECs on the cellular mechanical force detection device, and each microwell with a diameter of 200 microns accommodates about 20 endothelial cells to form cell aggregates. After one day of culture, unattached cells are removed, and the cellular mechanical force signal of vascular endothelial cell aggregates in each microwell is recorded.

S3. Adding lung cancer cell line A549 to the system, and starting real-time monitoring of changes in cellular mechanical force of vascular endothelial cells. This example not only orderly cultures fixed number of vascular endothelial cells in microwells, but also can identify the interaction between tumor cells and vascular endothelial cells by high-throughput monitoring of changes in cellular mechanical force.

Through this example, the influence of tumor cells on vascular endothelial cells can be effectively monitored and analyzed, providing a new experimental method and technical means for studying the angiogenesis process of tumors and their mechanism of action on vascular endothelial cells.

**Example 27: Method for Evaluating Effect of Sperm Cells on Egg Cells Through Cellular Mechanical Force**

This example aims to provide a method for evaluating the effect of sperm cells on egg cells based on cellular mechanical force detection, thereby providing a new technical means in the field of reproductive biology research. The method uses a cellular mechanical force detection device or characterization system, and a cellular mechanical force detection method to acquire cellular mechanical force information of cells or multicellular aggregates, and identifies the interaction between cells based on this. The specific operation steps of this example are as follows:

First, a structural film containing microwells with a diameter of about 30 microns is made of polydimethylsiloxane (PDMS) material, with a thickness of 5-10 microns. The PDMS microwell film is treated with Pluronic F127 solution, dried, and then laid on the microcolumn array of the cellular mechanical force detection device.

Subsequently, mouse egg cells are uniformly distributed on the above cellular mechanical force detection device, and each microwell with a diameter of 30 microns accommodates about 1 egg cell. These egg cells are cultured for two hours, then unattached cells are removed, and then cultured for another six hours to one day.

Thereafter, mouse sperm cells are added to the system to perform fertilization process, and real-time monitoring of changes in cellular mechanical force of egg cells is started. By precisely controlling each microwell to accommodate only one egg cell, this method can accurately observe and analyze the effect of sperm cells on a single egg cell, thereby providing an effective technical path for studying the mechanical interaction between cells during fertilization.

Through this example, fine monitoring of the interaction between sperm cells and egg cells can be realized, providing an important experimental basis for in-depth understanding of fertilization mechanism and early embryonic development. In addition, the use of this method may also include but not limited to optimization of human assisted reproductive technology and development of new drugs or therapeutic methods.

**Example** 28: **Method for Evaluating Interaction Result Between Cell Aggregates and Other Factors Through Cellular Mechanical Force** (Only Cellular Mechanical Force Magnitude, Partial Data with Labels, Partial Data Without Labels), Comprising the Following Steps:

S1. Acquiring cellular information of multiple cells of known different cells and/or multicellular aggregates, where some cells are cells of several known cell types or known cell states, and the remaining cells are cells of unknown cell types or unknown cell states; the cellular information is the magnitude of cellular mechanical force at a certain point in the cell acquired based on the cellular mechanical force detection device. This step specifically includes: using the cellular mechanical force detection device to collect information of the above several types of cells, which includes collecting cellular mechanical force magnitude information at multiple points of each cell, thereby acquiring multiple cellular mechanical force magnitude data at multiple points in multiple cells;

S2. Preprocessing the acquired cellular mechanical force magnitude information to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features, and P=1 here, that is, the cell feature is: cellular mechanical force magnitude;

S3. Using the above structured cellular information as input data to establish a cell feature model using semi-supervised machine learning and training the cell feature model using a large amount of structured cellular information of cells (including both labeled and unlabeled cells), and then applying the cell feature model to classification/clustering of unknown types or unknown states of cells.

In other embodiments similar to this example, optimization or improvement can be made in the following ways: for a single cell, further information processing is performed on the acquired cellular mechanical force magnitude information at multiple points, for example, calculating: average value of cellular mechanical force magnitude per unit area; distribution of cellular mechanical force magnitude in the cell; and other dimensional information, which can be added as new cell features to the two-dimensional feature matrix described in step S2, that is, expanding the content of P, and then subsequent machine learning is used to learn which features can better distinguish different drug-resistant cells and non-resistant cells.

**Example 29: Method for Evaluating Cells and/or Cell Aggregates Through Cellular Physical Information** (Magnitude and Direction of Cellular Mechanical Force, No Labels), Comprising the Following Steps:

S1. Acquiring cellular physical information, which is the magnitude and direction of cellular mechanical force at a certain point in the cell, and cell hardness acquired based on the cellular mechanical force detection device. Specifically including: using the cellular mechanical force detection device (nano-microcolumn sensor in this example) to collect cellular information of multiple cells, which includes collecting information of cellular mechanical force magnitude and direction at multiple points of each cell, thereby acquiring multiple cellular mechanical force magnitude and direction data at multiple points in multiple cells;

S2. Preprocessing the acquired information of cellular mechanical force magnitude and direction to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features, and P=2 here, that is, the cell features are: cellular mechanical force magnitude, cellular mechanical force direction;

S3. Using the above structured cellular information as input data to establish a cell feature model using unsupervised machine learning, and applying the cell feature model to clustering of unknown types or unknown states of cells.

Specifically, step S2 in this example processes cell information roughly as follows:
Assuming that n points of cellular mechanical force vector data (magnitude and direction) are obtained in a cell, these points are: ($i, i \in \{1,2 ... n\}$), corresponding to two two-dimensional coordinates : ($x_{t_0 i}$, $y_{t_0 i}$) and ($x_{t_n i}$, $y_{t_n i}$) respectively, where $t_0$ and $t_n$ correspond to the initial and displaced positions of the nano-microcolumns respectively.

In this way, ($x_{t_n i}$ - $x_{t_0 i}$, $y_{t_n i}$ - $y_{t_0 i}$) is the direction of force at each coordinate point. In addition, each coordinate point should also have scalar information, i.e., the magnitude $d$ of the force. Thus, the cell axis direction and center point coordinates can be estimated from existing data, and each cell can be organized into a vector of the same length based on this.

For example, based on each point in the cell, the center point can be calculated as: $\frac{1}{n}\sum_i(x_i, y_i)$. The cell axis is calculated by finding the two farthest points ($x_1$, $y_1$) and ($x_2$, $y_2$), and obtained using the following formula:

$$\beta = \frac{y_2 - y_1}{x_2 - x_1}, \alpha = y_2 - \beta x_2, y = \alpha + \beta x$$

.

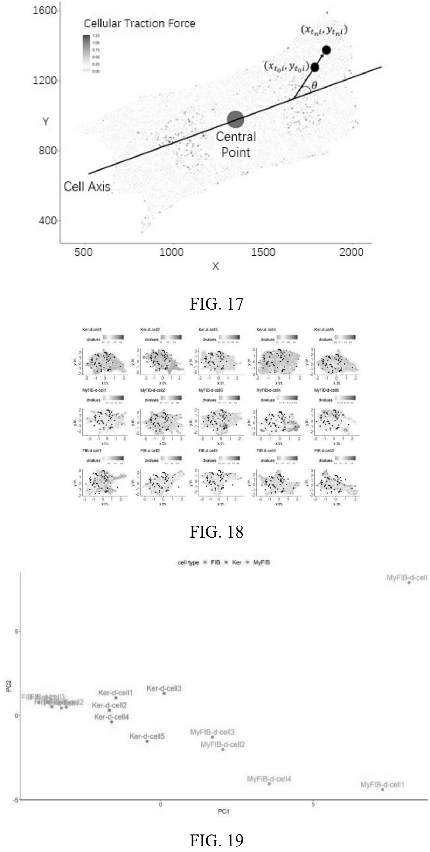

Please refer to FIG. 17, which is a schematic diagram of scalarization processing of displacement information at a certain point in the fourth example of the present invention. Each point in the figure represents a position, and the color depth of each point from light to dark indicates the force from small to large. After obtaining the cell axis of each cell, each point can be further quantified: calculate the angle $\theta$ between the displacement vector of each point and the cell axis. Each point can then be combined with the magnitude of the force (scalar), such as treating the magnitude d of the force as a weight, thereby processing each point into a scalar $s_i = \theta_i {}^* d_i$. In this way, the cell information of each cell can be organized into a vector $z = (s_1, ... s_n)$

In other embodiments similar to this example, optimization or improvement can be made in the following ways: For a single cell, further information processing is performed on the acquired multi-point cellular mechanical force magnitude or direction information, such as calculating: the average value of cellular mechanical force magnitude per unit area; the distribution of cellular mechanical force magnitude within the cell; the distribution of cellular mechanical force vectors within the cell; and other dimensional information. These can be used as new cell features and added to the two-dimensional feature matrix described in step S2, i.e., expanding the content of P, and then subsequent machine learning is used to determine which features can better distinguish different types or states of cells.

**Example 30: Method for Evaluating Cells or Cell Aggregates Through Cellular Mechanical Force** (Instantaneous Value of Cellular Mechanical Force Vector, Change of Cellular Mechanical Force Vector Within a Certain Time Interval, With Labels), Comprising the Following Steps:

S1. Acquiring cellular physical information of cells of different cell types or cell states (which may be known or unknown), where the cellular information is the instantaneous value of cellular mechanical force vector at a certain point in the cell and the change of cellular mechanical force vector at that point within a certain time interval acquired based on the cellular mechanical force detection device; specifically including: using the cellular mechanical force detection device to collect cellular information of multiple cells, which includes collecting information of cellular mechanical force magnitude and direction at multiple points of each cell, thereby acquiring multiple cellular mechanical force magnitude and direction data at multiple points in multiple cells;

S2. Preprocessing the acquired information of cellular mechanical force magnitude and direction to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features.

S3. Using the above structured cellular information as input data to establish a cell feature model using supervised machine learning and training the cell feature model using a large amount of structured cellular information of cells, and then applying the cell feature model to classification of unknown types or unknown states of cells (i.e., identification, "identification" in the present invention should be understood as broad sense identification, including both "classification", i.e., determining the type or state of cells; and "clustering", i.e., although the specific cell state or type is unknown, cells with the same or similar properties, possibly the same or similar types or states are clustered).

For example, in this example, a random forest (Random Forest, RF) algorithm is used to extract significant features and estimate model parameters, and then applied to new cells to estimate labels corresponding to new cells, that is, applying the cell feature model to classification of unknown types or unknown states of cells. In other embodiments, machine learning algorithms/ideas such as support vector machine (SVM) or deep learning may also be used to complete corresponding model establishment and training learning work.

In this example, since not only the instantaneous value of cellular mechanical force vector at a certain point in the cell is acquired, but also its interval within a certain time interval is included, the acquired mechanical data of a single cell can actually be analogous to an image (instantaneous value) or a video (multiple instantaneous values within a time dimension). Thus, if the dot matrix covered by the current cell is analogous to pixels in an image, and the information recorded at each point (multiple cell features such as force magnitude and direction) can be analogous to colors corresponding to pixels, machine learning algorithms in the field of image and video data processing can be used for subsequent machine learning, for example, machine learning widely used in image recognition, more precisely, convolutional neural network (CNN) in deep learning is used for data modeling and analysis.

**Example 31: Method for Evaluating Cells or Cell Aggregates Through Cellular Mechanical Force** (Instantaneous Value of Cellular Mechanical Force Vector, Change of Cellular Mechanical Force Vector Within a Certain Time Interval, With Labels), Comprising the Following Steps:

S1. Acquiring cellular physical information of cells of different cell types or cell states (which may be known or unknown), where the cellular information is the instantaneous value of cellular mechanical force vector at a certain point in the cell and the change of cellular mechanical force vector at that point within a certain time interval acquired based on the cellular mechanical force detection device; specifically including: using the cellular mechanical force detection device to collect cellular information of multiple cells, which includes collecting information of cellular mechanical force magnitude and direction at multiple points of each cell, thereby acquiring multiple cellular mechanical force magnitude and direction data at multiple points in multiple cells;

S2. Preprocessing the acquired information of cellular mechanical force magnitude and direction to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features.

S3. Using the above structured cellular information as input data to establish a cell feature model using supervised machine learning and training the cell feature model using a large amount of structured cellular information of cells, and then applying the cell feature model to classification of unknown types or unknown states of cells (i.e., identification, "identification" in the present invention should be understood as broad sense identification, including both "classification", i.e., determining the type or state of cells; and "clustering", i.e., although the specific cell state or type is unknown, cells with the same or similar properties, possibly the same or similar types or states are clustered). For example, in this example, a random forest (Random Forest, RF) algorithm is used to extract significant features and estimate model parameters, and then applied to new cells to estimate labels corresponding to new cells, that is, applying the cell feature model to classification of unknown types or unknown states of cells. In other examples, machine learning algorithms/ideas such as support vector machine (SVM) or deep learning may also be used to complete corresponding model establishment and training learning work.

In this example, since not only the instantaneous value of the cellular mechanical force vector at a certain point in the cell is acquired, but also its interval within a certain time interval is included, the acquired mechanical data of a single cell can actually be analogous to an image (instantaneous value) or a video (multiple instantaneous values within a time dimension). Thus, if the dot matrix covered by the current cell is analogous to pixels in an image, and the information recorded at each point (multiple cell features such as force magnitude and direction) can be analogous to colors corresponding to pixels, machine learning algorithms in the field of image and video data processing can be used for subsequent machine learning, for example, machine learning widely used in image recognition, more precisely, convolutional neural network (CNN) in deep learning is used for data modeling and analysis.

**Example 32: Method for Evaluating Interaction Between Cells or Cell Aggregates Through Cellular Physical Information** (Instantaneous Value of Cellular Mechanical Force Vector, Change of Cellular Mechanical Force Vector Within a Certain Time Interval, No Labels), Comprising the Following Steps:

S1. Acquiring cellular information, which is the instantaneous value of cellular mechanical force vector at a certain point in the cell and the change of cellular mechanical force vector at that point within a certain time interval acquired based on the cellular mechanical force detection device; specifically including: using the cellular mechanical force detection device to collect cellular information of multiple cells, which includes collecting information of cellular mechanical force magnitude and direction at multiple points of each cell, thereby acquiring multiple cellular mechanical force magnitude and direction data at multiple points in multiple cells;

S2. Preprocessing the acquired information of cellular mechanical force magnitude and direction to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features.

S3. Using the above structured cellular information as input data to establish a cell feature model using unsupervised machine learning, and applying the cell feature model to clustering of unknown types or unknown states of cells.

In this example, since not only the instantaneous value of cellular mechanical force vector at a certain point in the cell is acquired, but also its interval within a certain time interval is included, the acquired mechanical data of a single cell can actually be analogous to an image (instantaneous value) or a video (change within time dimension). Thus, if the dot matrix covered by the current cell is analogous to pixels in an image, and the information recorded at each point (multiple cell features such as force magnitude and direction) can be analogous to colors corresponding to pixels, machine learning algorithms in the field of image and video data processing can be used for subsequent machine learning, for example, machine learning widely used in image recognition, more precisely, convolutional neural network (CNN) in deep learning is used for data modeling and analysis.

**Example 33:** A Method for Evaluating Interaction Between Cells or Cell Aggregates Through Cellular Mechanical Force (Instantaneous Value of Cellular Mechanical Force Vector, Change of Cellular Mechanical Force Vector Within a Certain Time Interval, With Labels), Comprising the Following Steps:

S1. Acquiring cellular physical information of cells of different cell types or cell states (which may be known or unknown), where the cellular information is the instantaneous value of cellular mechanical force vector at a certain point in the cell and the change of cellular mechanical force vector at that point within a certain time interval acquired based on the cellular mechanical force detection device; specifically including: using the cellular mechanical force detection device to collect cellular information of multiple cells, which includes collecting information of cellular mechanical force magnitude and direction at multiple points of each cell, thereby acquiring multiple cellular mechanical force magnitude and direction data at multiple points in multiple cells;

S2. Preprocessing the acquired information of cellular mechanical force magnitude and direction to form structured cellular information; the structured cellular information includes the number of cells, the number of cell features, and feature information of each cell feature. At this time, the structured cellular information can be regarded as a two-dimensional matrix of $M_{N \times P}$ feature matrix, where N is the number of cells, P is the number of cell features.

S3. Using the above structured cellular information as input data to establish a cell feature model using supervised machine learning and training the cell feature model using a large amount of structured cellular information of cells, and then applying the cell feature model to classification of unknown types or unknown states of cells (i.e., identification, "identification" in the present invention should be understood as broad sense identification, including both "classification", i.e., determining the type or state of cells; and "clustering", i.e., although the specific cell state or type is unknown, cells with the same or similar properties, possibly the same or similar types or states are clustered). For example, in this example, a random forest (Random Forest, RF) algorithm is used to extract significant features and estimate model parameters, and then applied to new cells to estimate labels corresponding to new cells, that is, applying the cell feature model to classification of unknown types or unknown states of cells. In other embodiments, machine learning algorithms/ideas such as support vector machine (SVM) or deep learning may also be used to complete corresponding model establishment and training learning work.

In this example, since not only the instantaneous value of cellular mechanical force vector at a certain point in the cell is acquired, but also its interval within a certain time interval is included, the acquired mechanical data of a single cell can actually be analogous to an image (instantaneous value) or a video (change within time dimension). Thus, if the dot matrix covered by the current cell is analogous to pixels in an image, and the information recorded at each point (multiple cell features such as force magnitude and direction) can be analogous to colors corresponding to pixels, machine learning algorithms in the field of image and video data processing can be used for subsequent machine learning, for example, machine learning widely used in image recognition, more precisely, convolutional neural network (CNN) in deep learning is used for data modeling and analysis.

**Example 34: This Example Provides a Cellular Physical Characterization Device and System**

As shown in FIG. 1, this example provides a cellular physical characterization device, which uses one of light reflection layer and magnetic material or a magnetic metal light reflection layer to affect the intensity of light reflection signal to realize measurement of multimodal biophysical information of cells.

The top of the microcolumn 12 has a coating of magnetic metal (such as iron, cobalt, nickel, etc.) (in other examples, other magnetic materials can be used instead, and the position where the magnetic material is set can be the side or inside of the microcolumn, as long as the microcolumn can generate magnetic force under magnetic field). This coating with magnetic metal can serve as the light reflection layer 105. In a preferred embodiment, the top of the microcolumn 12 is provided with a magnetic metal light reflection layer.

It should be reminded that the term "coating" is used in this example only to indicate that the light reflection layer 13 in this example can be prepared by a coating process, but does not limit that the light reflection layer 105 must be prepared by a coating process, for example, it can also be prepared by sputtering or evaporation process.

The characterization system is composed of one of the above characterization devices, a light signal emitting device, a light signal detecting device and a magnetic field emitting device. Wherein, the light signal emitting device is used to emit predetermined light, the light signal detecting device is used to detect light reflected from the light reflection layer 13, and the magnetic field emitting device is used to emit a magnetic field to generate magnetic force with the magnetic metal. The light emitted by the light signal emitting device irradiates the light reflection layer 13 through the incident light path, and the light reflected by the light reflection layer 13 enters the light signal detecting device through the reflected light path.

When measuring multimodal biophysical characteristics of cells using the above cellular physical characterization system, the specific operation steps are:

S1. Culturing the cell to be measured (characterized) on the cellular physical characterization device shown in FIG. 1, and after one to two days of culture, the cell to be measured (characterized) is completely attached and grown on the physical characterization device;

S2. Emitting predetermined light by the light signal emitting device;

S3. Detecting the light acted by the cellular physical characterization device by the light signal detecting device. The "action" in this example may be only the reflection of predetermined light by the light reflection layer provided on any one of the top and side of the microcolumn of the cellular physical characterization device, or the reflection of predetermined light by the light reflection layers provided on both the top and side of the microcolumn. Preferably, it may also be that the magnetic metal light reflection layer on the top of the microcolumn tilts, pierces into the cell to be characterized, deforms, pulls or swings under the magnetic force of a specific direction and specific intensity emitted by the magnetic field emitting device, and at the same time, the magnetic metal light reflection layer reflects light during microcolumn deformation. Therefore, the system of this example can separately measure the mechanical force of the cell to be characterized, or separately measure the hardness of the cell to be characterized, or jointly measure the mechanical force and hardness of the cell to be characterized.

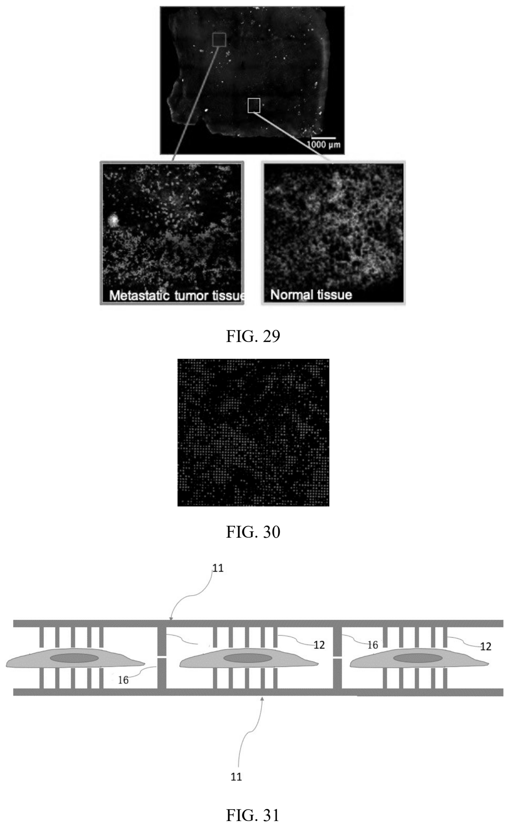

As shown in FIG. 31, in other specific embodiments, two cellular physical characterization devices provided in the present application are oppositely arranged to form a double-sided structure, with the base on the outside and the inner side enclosing a three-dimensional accommodating cavity (sandwich structure) for cells or multicellular aggregates. The volume or height of the three-dimensional accommodating cavity can be adjusted according to actual needs. For example, when the cell to be characterized is the opposite side of a single cell, the height of the three-dimensional accommodating cavity can be controlled between 5nm-2mm. When the cell to be characterized is a living tissue, the height and volume can be adjusted to be larger, and the height can even reach 5cm, and the volume can reach 30cm3.

**Example 35: Using Cellular Mechanical Force Chip to Monitor Organoid Attachment and Evaluate Response to Chemotherapeutic Drugs and Immune Cell Attack**

This example provides an immune-organoid chip. By monitoring changes in cellular mechanical force of organoids on the chip, the response of organoids to chemotherapeutic drugs and immune cell attack is evaluated.

S1. Preparing the chip: selecting a cellular mechanical force chip with microcolumn structure, coating the top of the microcolumn with a suitable extracellular matrix (ECM) to promote organoid attachment and growth. Using anti-adhesion treatment on the sides of microcolumns and between microcolumns.

S2. Seeding organoids: placing the cultured organoids on the mechanical force chip. Ensure that the organoids are in contact with the chip surface so that the organoids attach to the chip. Cells and corresponding extracellular matrix substances can also be directly added to the chip to directly culture organoids and monitor mechanical response and changes during organoid development.

S3. Monitoring organoid attachment: real-time monitoring the attachment of organoids on the mechanical force chip using light reflection signals, and observing changes in cellular mechanical force.

S4. Adding chemotherapeutic drugs: adding an appropriate amount of chemotherapeutic drugs to the organoid culture medium.

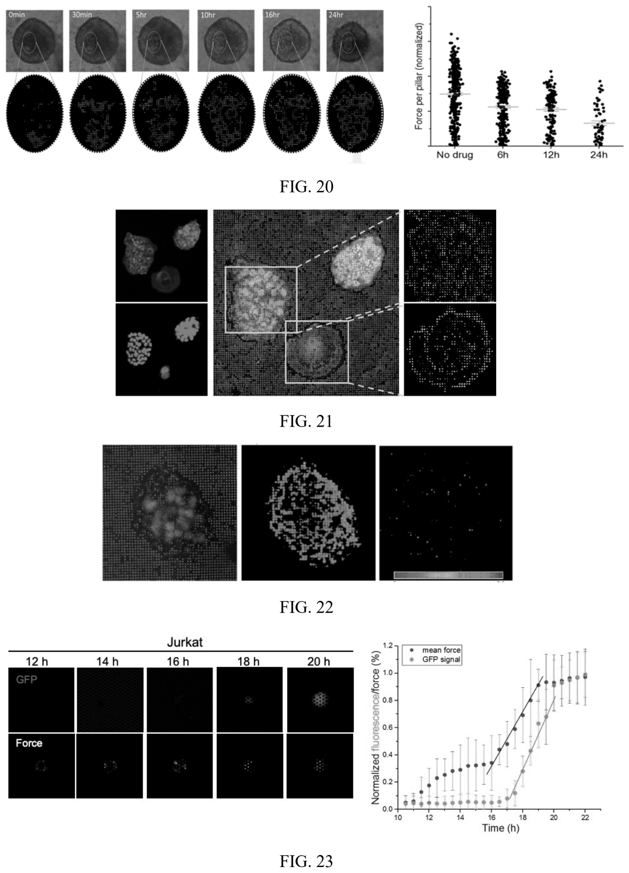

S5. Monitoring organoid response to drugs: continuing to monitor changes in cellular mechanical force and hardness of organoids under drug action using light reflection signals and microcolumn deflection. Observing the reduction of organoid viability under drug action (FIG. 20) or adding immune cells (NK cells or T cells) for co-culture to monitor changes in organoid viability.

**Example 36: Using Cellular Mechanical Force Chip to Characterize Heterogeneous Tumor Spheroids and Screen for Drug-Resistant or Immune-Evasive Individuals**

This example provides an immune-organoid chip. By monitoring changes in cellular mechanical force on heterogeneous tumor spheroids, individuals with drug resistance or immune evasion are screened.

S1. Preparing the chip: selecting a cellular mechanical force chip with microcolumn structure, coating the top of the microcolumn with a suitable extracellular matrix (ECM) to promote attachment and growth of tumor spheroids. Using anti-adhesion treatment on the sides of microcolumns and between microcolumns.

S2. Culturing heterogeneous tumor spheroids: separately culturing tumor spheroids from different sources or with different drug resistance characteristics in the laboratory, and using a restrictive structure to constrain the size and shape of the spheroids (FIG. 22). Tumor spheroids can also be extracted and isolated from patients.

S3. Seeding tumor spheroids: placing the cultured heterogeneous tumor spheroids on the mechanical force chip. Ensure that the tumor spheroids are in contact with the chip surface so that the tumor spheroids attach to the chip.

S4. Monitoring tumor spheroid attachment: real-time monitoring the attachment of tumor spheroids on the mechanical force chip using light reflection signals, and observing changes in cellular mechanical force.

S5. Adding chemotherapeutic drugs: adding an appropriate amount of chemotherapeutic drugs to the tumor spheroid culture medium. Or adding immune cells for co-culture.

S6. Monitoring tumor spheroid response to drugs: continuing to monitor changes in cellular mechanical force and hardness of tumor spheroids under drug action using light reflection signals and microcolumn deflection. Observing the growth inhibition of different tumor spheroids under drug action to screen for individuals with drug resistance characteristics (FIG. 21). The killing effect of immune cells on tumor spheroids and the effect of drugs on immune cells can also be obtained to evaluate the targeting of drugs.

Summary: This example demonstrates how to use cellular mechanical force chips to characterize heterogeneous tumor spheroids and screen for drug-resistant individuals. By monitoring changes in cellular mechanical force, important information can be provided for drug resistance research and personalized treatment.

**Example 37: Using Cellular Mechanical Force Chip to Characterize Immune-Tumor Cell Interaction and Evaluate Drug Intervention Effect**

This example provides an immune-tumor chip. By monitoring the relationship between mechanical characteristics of tumor cells and immune cell interaction, and the influence of drug intervention on this relationship.

S1. Preparing the chip: selecting a cellular mechanical force chip with microcolumn structure, coating the top of the microcolumn with a suitable extracellular matrix (ECM) to promote attachment and growth of tumor cells and immune cells. Using anti-adhesion treatment on the sides of microcolumns and between microcolumns.

S2. Seeding tumor cells and immune cells: separately seeding tumor cells and immune cells (such as NK cells or T cells) on the mechanical force chip to allow them to interact.

S3. Monitoring mechanical characteristics of tumor cells: real-time monitoring changes in cellular mechanical force and hardness of tumor cells during interaction with immune cells using light reflection signals and microcolumn deflection. At the same time, guiding microcolumn deflection through magnetic field to measure cell hardness.

S4. Observing killing effect of immune cells on tumor cells: it is found that the mechanical force of tumor cells is positively correlated with hardness, and softer tumor cells (smaller cellular mechanical force) are difficult to be recognized and killed by immune cells.

S5. Drug intervention: adding drugs that harden tumor cells to the culture medium, and observing changes in cellular mechanical force and hardness of tumor cells.

S6. Evaluating drug intervention effect: under drug action, tumor cells harden and cellular mechanical force increases. It is observed that the killing effect of immune cells on tumor cells is enhanced accordingly.

Summary: This example demonstrates how to use cellular mechanical force chips to characterize immune-tumor cell interaction and the influence of drug intervention on this relationship. In particular, the positive correlation between cellular mechanical force and cell hardness indicates the importance of characterizing these two properties simultaneously. By studying the mechanical characteristics of tumor cells, it can provide optimization for immunotherapy strategies.

The cellular mechanical force chip referred to in the present invention can be the cellular mechanical force retrieval device, cell/multicellular aggregate interaction characterization and identification system disclosed in the present invention. Or other cellular mechanical force chips combined with light signal reflection through microcolumns.

**Example 38: Method for Evaluating Interaction Between Expressed Proteins, Polypeptides and Cells/Cell Aggregates Through Cellular Mechanical Force**

This example provides acquiring cellular mechanical force information of cells/multicellular aggregates through the cellular mechanical force detection device or cell/cell aggregate characterization system described in any one of the above examples or the cellular mechanical force detection method described in any one of the above schemes; identifying the interaction between substances and cells or/and multicellular aggregates according to the cellular mechanical force information: using mechanical force information of transfected cells to predict transfection status of to be tested transfected cells. According to the transfection status, optionally, combined with microfluidic microfluidics, high-throughput biological screening can be realized. This example can be applied to discover that there are significant differences in cellular mechanical force and hardness characteristics between successfully transfected cells and unsuccessfully transfected cells, and it is first discovered that there are significant differences in cellular mechanical force and hardness characteristics between cells expressing and not expressing biologically active compounds (including proteins, polypeptides) in cells. According to these physical characteristics combined with artificial intelligence training recognition models, successfully transfected cells and high-yield cells expressing biologically active compounds can be quickly identified non-invasively and without labels, and combined with microfluidic microfluidics to realize high-throughput biological screening.

Experimental verification shows that: when added to the culture system, untransfected cells may not be killed because the drug concentration is too low or the cell density is too high. Rapidly dividing cells are more likely to be killed than slowly proliferating cells. Control cells (untransfected) may be killed 5-7 days after adding antibiotics, and clones of transfected cells (resistant clones) need 10-14 days to form.

In some specific examples, the purpose of transfection is to change cell morphology or function, such as affecting cytoskeleton, cell adhesion or cell differentiation, etc., then successfully transfected cells may show different mechanical force patterns and magnitudes from untransfected or unsuccessfully transfected cells. In some specific examples, the purpose of transfection is to change gene expression or protein level of cells without directly affecting cell morphology or function, then there may be no significant difference between successfully and unsuccessfully transfected cells. In some specific examples, the transfection method uses viral vectors or other physical means instead of chemical reagents such as liposomes, then the transfection process itself may cause certain damage or stress response to cells, thereby affecting cellular mechanical force.

The present invention first discovers that there is a significant change in cellular mechanical force between successfully transfected cells and unsuccessfully transfected cells. The system of the present invention has real-time characterization, fast speed, can real-time monitor cell status and potential cytotoxicity during the experiment, and optimize transfection plan in time.

**Example 39: Method and Use for Characterization, Classification and Identification of Interaction Between Expressed Fat and Cells/Cell Aggregates Through Cellular Mechanical Force**

This example provides acquiring cellular physical information of cells/multicellular aggregates through the cellular mechanical force detection device or cell/cell aggregate characterization system described in any one of the above examples or the cellular mechanical force detection method described in any one of the above schemes; identifying the interaction between fat and cells or/and multicellular aggregates according to the cellular physical information: adipocytes are the largest connective tissue cells in the human body, and their main function is to store and mobilize lipids. Adipocytes can be divided into two categories: white adipocytes and brown adipocytes, which have great differences in morphology, function and origin. White adipocytes are unilocular, that is, a single white adipocyte contains a large triglyceride-rich lipid droplet, which occupies 90% of the cell volume, squeezing other components and nuclei to the edge. White adipose tissue is mainly responsible for storing energy and releasing free fatty acids for use by other tissues when needed. White adipose tissue can also secrete a variety of hormones and factors, such as leptin, angiotensin, vascular endothelial growth factor, etc., to regulate blood pressure, appetite, glucose metabolism and other physiological processes. Brown adipocytes are multilocular, that is, a single brown adipocyte contains many small triglyceride-rich lipid droplets, and contains highly condensed mitochondria, making them appear brown. Brown adipose tissue is mainly responsible for generating heat and consuming excess energy when cold or overeating. Brown adipose tissue can also secrete some hormones and factors with different or opposite effects to white adipose tissue, such as hepcidin, neuron guidance factor, etc., to regulate iron metabolism, neurogenesis and other physiological processes. For different types of adipocytes, they each play normal and important roles in healthy state; but in unhealthy state, such as overnutrition or malnutrition, they may undergo some abnormal changes or transformations, and affect human health. For example, in overnutrition, white adipose tissue will increase in number and size, leading to metabolic disorders, diabetes, cardiovascular diseases, etc.; while brown adipose tissue will decrease in activity or transform into white-like characteristics, and reduce energy consumption rate.

The inventor discovery that there are obvious dynamic changes in cellular mechanical force during adipocyte differentiation, which can be used to real-time monitor the conversion process of white adipose, beige adipose and brown adipose. And provide a high-throughput testing platform for related drug testing. The cellular mechanical force detection device of the present invention can be used to monitor obvious dynamic changes in cellular mechanical force during adipocyte differentiation, which can be used to real-time monitor the conversion process of white adipose, beige adipose and brown adipose. White adipocytes and brown adipocytes have obvious differences in morphology and function, which are also reflected in their cellular mechanical force. Generally speaking, white adipocytes are harder than brown adipocytes, but produce smaller mechanical force. Cellular mechanical force is closely related to cell differentiation, and some signaling pathways such as Rho/ROCK pathway can regulate the process of adipocyte precursor cells differentiating into white or brown adipose. In some specific examples, by changing the stiffness of the culture substrate or applying periodic stretching, the differentiation direction and rate of adipocyte precursor cells can be affected, and characteristic markers after differentiation of different types of adipose can be detected. In some specific examples, some drugs such as tea polyphenols (epigallocatechin gallate) can inhibit white adipose differentiation or promote brown adipose differentiation by affecting Rho/ROCK pathway or other signaling pathways, and these effects can also be observed by mechanical force microscopy.

This example can effectively study differences in morphology, function and metabolism between different types of adipocytes, and can screen out some drug candidates that are beneficial for preventing and treating obesity and related diseases.

**Example 40: Method for Evaluating Interaction Between Other Substances and Cells/Cell Aggregates Through Cellular Mechanical Force**

This example provides acquiring cellular physical information of cells/multicellular aggregates through the cellular mechanical force detection device or cell/cell aggregate characterization system described in any one of the above examples or the cellular mechanical force detection method described in any one of the above schemes; evaluating the interaction between substances and cells or/and multicellular aggregates according to the cellular physical information: In some specific examples, in real-time high-throughput monitoring of cellular mechanical force, in application of synthetic biology, combined with semi-solid medium and antibodies or secondary antibodies, to effectively verify successfully transfected or high-yield cell lines. The structure can be used for machine learning. In some specific examples, in application of synthetic biology, it can monitor influence of genetic engineering (such as transfection) on cells, detect small clone clusters or single cells near large clones, and ensure monoclonal isolation of target clones. Structural and morphological parameters of clones (such as sphericity and shape) can be defined and suspicious (such as elongated) clones can be excluded. In some specific examples, in application of synthetic biology, the system can help establish an optimal dose-response curve (dose-response curve or kill curve) by monitoring cell viability and state to determine the minimum effective concentration of drug to kill non-resistant cells. At the same time, real-time monitoring of cells during drug screening to adjust experimental plan in time. In some specific examples, in addition to single cells, it can also real-time monitor the state and dynamic changes of multicellular aggregates (including tumor spheroids, organoids, living tissues, etc.) with high throughput and low cost. In some specific examples, according to different types of mechanical characteristics and change rules combined with artificial intelligence training recognition models, further judge or predict type, state, behavior and differentiation direction of single or multicellular aggregates. In some specific examples, the present invention is applicable to synthetic biology, diagnosis, drug discovery, early tumor screening, cell therapy, precision medicine and other fields. In some specific examples, it is judged whether the measured cells grow alone or aggregate into aggregates during culture; the characterization and identification system and method of the present invention have real-time characterization, fast speed, can real-time monitor cell state and potential cytotoxicity during the experiment, and optimize transfection plan in time; in some specific examples, it can be used for establishment of high-yield stable cell lines, which is crucial for large-scale production of recombinant proteins and antibody drugs. For currently widely used CHO cells, multiple subtype cell banks have been established, which can efficiently develop stable high-yield expression cell lines and improve development speed. In some specific examples, it can be used for microscopic images (before and after capture) for quality control and real-time images during capture. Great progress has been made in preparing protein drugs using mammalian cells, and with the rapid development of biotechnology and synthetic biology, many genetically engineered drugs and recombinant drugs have been synthesized, and many new drugs have been discovered, enriching the types of protein drugs and increasing the chance of curing diseases. However, currently 70% of protein drugs are produced by Chinese hamster ovary cells CHO, and the long growth cycle of intracellularly expressed protein drugs leads to slow screening process; the present invention establishes a dose-response curve (dose-response curve or kill curve) to determine the minimum effective concentration to kill non-resistant cells. In some specific examples, it can be applied to flow cytometry: fluorescently labeled antibodies or fluorescent probes can be used to label expression of target proteins in the cells/cell aggregates, and then flow cytometry can be used to analyze and sort cells. In some specific examples, drug development usually relies on high-throughput cell-based screening of large compound libraries. However, due to lack of chemical miniaturization and parallelization methods, and strict separation from biological screening and non-synthesis of bioactive compounds, this method is expensive and inefficient.

The characterization system, identification system and method of the present invention show an on-chip platform that combines solution-based compound library synthesis with high-throughput biological screening (chemBIOS). In some specific examples, the inventor first time discovery that there are significant differences in cellular mechanical force characteristics between cells with and without intracellular expression of bioactive compounds (including proteins, polypeptides), and high-expression clones can be screened and isolated through cellular mechanical force characteristics; in some specific examples, in the process of studying the interaction between cells and macromolecules, it is first time discovery that characterization method through cellular force is more sensitive than traditional gene or proteomics characterization methods, and can detect changes in cells faster and more sensitively. In some specific examples, in addition to single cells, it can also be applied to real-time monitoring of state and dynamic changes of multicellular aggregates (including tumor spheroids, organoids, living tissues, etc.) with high throughput and low cost. In some specific examples, applications in synthetic biology refers to using genetic engineering or other means to transformation or create biological systems with specific functions, such as efficiently synthesizing certain metabolites or drugs. In some specific examples, in application in synthetic biology, target genes are usually introduced into host cells through transfection, transformation or other methods, and high-expression cell lines are screened to improve production efficiency.

The principle of identifying and sorting high-expression cell lines by characterization of cellular mechanical force is: expression of target genes may affect cell morphology or function, thereby changing interaction force between cells and matrix. For example, if the target gene encodes a protein that can change cytoskeleton, adhesion or differentiation state, cells with high expression of this gene may have obvious differences in mechanical force patterns and magnitudes from low-expression or untransfected cells. When using mechanical force microscopy to identify and sort high-expression, other methods such as fluorescence detection, enzyme-linked immunosorbent assay can be combined to verify results, further improving accuracy of acquired cellular physical information and prediction accuracy.

## Example 41:

This example provides a method for acquiring cellular physical information of cells/multicellular aggregates using the cellular mechanical force detection device, cell/cell aggregate characterization system, or cellular mechanical force detection method described in any of the above examples; and identifying interactions between macromolecules (e.g., proteins, sugar molecules, lipids) or microorganisms and cells and/or multicellular aggregates based on the cellular physical information:

In some specific examples, comparing the effects of different extracellular matrices on cellular mechanical force, the steps are:

S1. Cover the top of microcolumns of the cellular mechanical force detection device with different extracellular matrices (e.g., collagen, fibronectin, and laminin) respectively, or print extracellular matrices on the microcolumn tops in specific patterns using microcontact printing; treat the sides of microcolumns and the spaces between microcolumns with Pluronic F127 for anti-adhesion.

S2. Culture lung cancer cell line A549 on cellular mechanical force detection devices coated with different extracellular matrices respectively.

S3. Real-time monitor cellular mechanical force, and compare the strength and distribution of cellular mechanical force on different extracellular matrix coatings.

In some specific examples, a method for detecting the effect of antibodies on cellular mechanical force, the steps are:

S1. Cover the top of microcolumns of the cellular mechanical force detection device with CD3 antibodies; treat the sides of microcolumns and the spaces between microcolumns with Pluronic F127 for anti-adhesion.

S2. Add NFAT reporter (eGFP) Jurkat recombinant cell line; CD3 antibodies will attract T cells to contact the chip, and this T cell line will express green fluorescent protein when activated by CD3 antibodies.

S3. Real-time monitor changes in cellular mechanical force and expression of green fluorescent protein as shown in FIG. 23.

In some specific examples, a method for detecting the effect of sugar molecules (glycoproteins or glycolipids) on cellular mechanical force:

S1. Cover the top of microcolumns of the cellular mechanical force detection device with Lipopolysaccharides (LPS); treat the sides of microcolumns and the spaces between microcolumns with Pluronic F127 for anti-adhesion.

S2. Add THP1-ASC-GFP cell lines; LPS will attract cells to contact the chip, and this monocyte cell line will express green fluorescent protein when activated by LPS.

S3. Real-time monitor changes in cellular mechanical force and expression of green fluorescent protein.

In some specific examples, a method for detecting the effect of proteins on cellular mechanical force:

S1. Cover the top of microcolumns of the cellular mechanical force detection device with zymosan; treat the sides of microcolumns and the spaces between microcolumns with Pluronic F127 for anti-adhesion.

S2. Add THP1-ASC-GFP cell lines; zymosan will attract cells to contact the chip, and this monocyte cell line will express green fluorescent protein when activated by zymosan. S3. Real-time monitor changes in cellular mechanical force and expression of green fluorescent protein.

In some specific examples, a method for detecting the effect of microorganisms on cellular mechanical force:

S1. Cover the top of microcolumns of the cellular mechanical force detection device with paraformaldehyde-treated bacteria; treat the sides of microcolumns and the spaces between microcolumns with Pluronic F127 for anti-adhesion.

S2. Add THP1-ASC-GFP cell lines; bacteria will attract cells to contact the chip, and this monocyte cell line will express green fluorescent protein when activated by bacteria.

S3. Real-time monitor changes in cellular mechanical force and expression of green fluorescent protein.

**Example 42: Method for Measuring Immune Cell-Molecule Interactions and Cell Activation Process by Characterizing Cellular Mechanical Force and Stiffness**

This example provides a method for evaluating immune cell-molecule interactions (e.g., T cells with CD3 antibodies) and cell activation processes by measuring changes in immune cell mechanical force and stiffness. The expression of green fluorescent protein is used as a marker of cell activation, cross-validated with measurements of cellular mechanical force and stiffness.

S1. Prepare a cellular mechanical force detection device with microcolumn tops coated with CD3 antibodies; treat the sides of microcolumns and the spaces between microcolumns with Pluronic F127 for anti-adhesion.

S2. Add NFAT reporter (eGFP) Jurkat recombinant cell line to the chip; CD3 antibodies will attract T cells to contact the chip, and this T cell line will express green fluorescent protein when activated by CD3 antibodies.

S3. Real-time monitor changes in cellular mechanical force and expression of green fluorescent protein. By analyzing changes in cellular mechanical force at different time points, study immune cell-molecule interactions and cell activation processes.

S4. Use a pressure transmission device to insert microcolumns into cells, apply a magnetic field, and characterize cell stiffness by detecting light reflection signals.

S5. Analyze experimental data to monitor changes in cellular mechanical force and stiffness during immune cell-molecule interactions and activation. Simultaneously, observe green fluorescent protein expression and cross-validate with mechanical measurements to evaluate cell activity under different activation conditions.

## Example 43: Method for Distinguishing Activated and Non-Activated T Cells and Determining Their Activation Ratio by Measuring Cellular Mechanical Force and Stiffness

This example provides a method for distinguishing activated and non-activated T cells and determining their activation ratio by measuring cellular mechanical force and stiffness. This method can be used to characterize cell therapy samples and predict the success probability of cell therapy.

S1. Culture T cell line NFAT reporter (eGFP) Jurkat cells on a cellular mechanical force detection device; control the amount of culture medium to ensure T cells contact the device, and measure the mechanical force of non-activated cells.

S2. Add CD3 antibodies, CD28 antibodies, and cytokines to the culture medium to activate T cells; when T cells are activated by antibodies, they will express green fluorescent protein.

S3. Real-time monitor changes in mechanical force and green fluorescent protein expression of each T cell.

S4. Use a pressure transmission device to insert microcolumns into cells, apply a magnetic field, and characterize cell stiffness by detecting light reflection signals.

S5. Observe that cellular mechanical force gradually increases when T cells are activated, showing a positive correlation with green fluorescence.

S6. Input changes in cellular mechanical force and stiffness during each T cell activation process into a database, and use machine learning to establish a model for determining T cell activation levels based on mechanical force, stiffness characteristics, spatial distribution, and temporal dynamics.

S7. Isolate T cells from donor peripheral blood mononuclear cells (PBMC) using CD4 and CD8 antibodies.

S8. Culture the isolated T cell pool on a cellular mechanical force detection device; control the amount of culture medium to ensure all T cells contact the device.

S9. Record the distribution of mechanical force magnitudes of all T cells, use the previously established model for analysis, and determine the ratio of activated to non-activated T cells in the donor. This value can be used to predict the success rate of cell therapy.

## Example 44: Technology for In Vitro Culture Monitoring of CAR-T Cell Therapy Using Microfluidics and Cellular Mechanical Force Detection Device

This example provides a technology for in vitro culture monitoring of CAR-T cell therapy using microfluidics and cellular mechanical force detection device, to more effectively monitor and evaluate the growth, activation, and efficacy of CAR-T cells in vitro.

S1. Cover the top of microcolumns of the characterization device chip with CD3 and CD28 antibodies; treat the sides of microcolumns and the spaces between microcolumns with Pluronic F127 for anti-adhesion.

S2. Place the chip at the bottom of a microfluidic device.

S3. Inject T cells isolated from donor peripheral blood mononuclear cells (PBMCs) into the microfluidic device and activate T cells. Simultaneously, determine whether all T cells are fully activated by monitoring cellular mechanical force.

S4. After complete activation of T cells, add lentivirus for transduction to enable T cells to express chimeric antigen receptors with antibody-specific sequences (sc-fv) that recognize cancer cell surface proteins.

S5. Remove transduced T cells (CAR-T) from the microfluidic device and continue culture expansion.

S6. Place a PDMS microwell membrane on the cellular mechanical force detection device, and culture tumor cells, tumor multicellular spheroids, or tumor organoids on the microwell-equipped detection device; cells will grow orderly in the microwells.

S7. Add CAR-T cells to the cellular mechanical force detection device containing tumor cells, and real-time monitor tumor cell mechanical force. When CAR-T cells effectively kill tumor cells, tumor cellular mechanical force will significantly decrease.

This example enables more effective monitoring and evaluation of the mechanical properties of CAR-T cells during activation, expansion, and tumor cell killing in vitro, providing more accurate data support for CAR-T cell therapy.

**Example 45: Technology for In Vitro Culture Monitoring of TCR-T Cell Therapy Using Microfluidics and Cellular Mechanical Force Detection Device**

This example provides a technology for in vitro culture monitoring of TCR-T cell therapy using microfluidics and cellular mechanical force detection device, to more effectively monitor and evaluate the growth, activation, and efficacy of TCR-T cells in vitro.

S1. Cover the top of microcolumns of the characterization device with CD3 and CD28 antibodies; treat the sides of microcolumns and the spaces between microcolumns with Pluronic F127 for anti-adhesion.
S2. Place the characterization device at the bottom of a microfluidic device.
S3. Inject T cells isolated from donor peripheral blood mononuclear cells (PBMCs) into the microfluidic device and activate T cells. Simultaneously, determine whether all T cells are fully activated by monitoring cellular mechanical force.
S4. After complete activation of T cells, use CRISPR-Cas9 technology to modify T cell receptors (TCR) to express sequences that recognize cancer cell antigens.
S5. Remove modified TCR-T cells from the microfluidic device and continue culture expansion.
S6. Place a PDMS microwell membrane on the cellular mechanical force detection device, and culture tumor cells, tumor multicellular spheroids, or tumor organoids on the microwell-equipped detection device; cells will grow orderly in the microwells.
S7. Add TCR-T cells to the cellular mechanical force detection device containing tumor cells, and real-time monitor tumor cell mechanical force. When TCR-T cells effectively kill tumor cells, tumor cellular mechanical force will significantly decrease.

This example enables more effective monitoring and evaluation of the mechanical properties of TCR-T cells during activation, expansion, and tumor cell killing in vitro, providing more accurate data support for TCR-T cell therapy.

**Example 46: Using Characterization System to Monitor Changes in Cellular Mechanical Force for Evaluating Antibody-Drug Conjugate Efficacy in Treating Suspension Cancers**

This example provides a method for evaluating the therapeutic efficacy of antibody-drug conjugates (ADCs) in treating suspension cancers by monitoring changes in cellular mechanical force using a characterization system.

S1. Cover the top of microcolumns of the characterization system with antibody-drug conjugates; treat the sides of microcolumns and spaces between microcolumns with Pluronic F127 for anti-adhesion.
S2. Culture blood cancer cells on this device; the antibody coating will attract blood cancer cells to contact the device.
S3. Real-time monitor cellular mechanical force of blood cancer cells by detecting light reflection signal intensity. When antibody-drug conjugates effectively inhibit cell growth or induce apoptosis, cellular mechanical force will significantly decrease.

Monitoring changes in cellular mechanical force using the characterization system enables better understanding of ADC therapeutic efficacy for suspension cancers. Real-time mechanical force monitoring helps evaluate ADC efficacy, thereby optimizing drug design and treatment protocols.

**Example 47: Using Characterization System to Monitor Changes in Cellular Mechanical Force and Stiffness for Evaluating Antibody-Drug Conjugate Efficacy in Treating Adherent Cancers**

This example provides a method for evaluating antibody-drug conjugate efficacy in treating adherent cancers by monitoring changes in cellular mechanical force and stiffness.

S1. Cover the top of microcolumns of the characterization system with extracellular matrix; treat microcolumn sides, spaces between microcolumns, and the base with Pluronic F127 for anti-adhesion.

S2. Culture lung cancer cells on the characterization system for one day to ensure complete attachment to the device.
S3. Add antibody-drug conjugates to the culture medium.
S4. Real-time monitor cellular mechanical force and stiffness of lung cancer cells by detecting light reflection signal intensity. When antibody-drug conjugates effectively inhibit cell growth or induce apoptosis, cellular mechanical force and stiffness will significantly decrease.

Monitoring changes in cellular mechanical force and stiffness via the characterization system provides important basis for evaluating ADC therapeutic efficacy in treating adherent cancers.

**Example 48: Evaluating Antibody-Drug Conjugate Efficacy by Monitoring Cellular Mechanical Force Changes in Living Tissues**

This example aims to provide a method for evaluating antibody-drug conjugate therapeutic efficacy on tumor cells by monitoring cellular mechanical force changes in living tissues.

S1. Cover the top of microcolumns of the characterization system with extracellular matrix; treat microcolumn sides and spaces between microcolumns with Pluronic F127 for anti-adhesion.

S2. Use a tissue sectioning machine to cut fresh tumor tissue into 200-micrometer-thick slices.

S3. Place living tissue slices on the cellular mechanical force detection device and culture for one day to ensure complete attachment to the chip. Tumor cell regions are known to have stronger mechanical force than non-tumor regions.

S4. Add antibody-drug conjugates to the culture medium.

S5. Real-time monitor cellular mechanical force in tumor and non-tumor cell regions by detecting light reflection signal intensity. When antibody-drug conjugates effectively inhibit cell growth or induce apoptosis, cellular mechanical force will significantly decrease.

S6. Additionally, by comparing mechanical force changes between tumor and non-tumor regions, determine whether antibody-drug conjugates specifically kill tumor cells and evaluate effects on normal cells.

This example provides important basis for evaluating ADC therapeutic efficacy on tumor cells by monitoring cellular mechanical force changes in living tissues.

**Example 49: Evaluating Antibody-Drug Conjugate Efficacy by Monitoring Cellular Mechanical Changes in Multicellular Spheroids**

This example aims to provide a method for evaluating antibody-drug conjugate efficacy by monitoring cellular mechanical changes in multicellular spheroids.

S1. Prepare the characterization system with microcolumn tops coated with extracellular matrix; treat microcolumn sides and spaces between microcolumns with Pluronic F127 for anti-adhesion.
S2. Culture tumor cells as multicellular spheroids on this chip for one day to ensure complete attachment. Add antibody-drug conjugates to the culture medium.
S3. Real-time monitor cellular mechanical changes in multicellular spheroids by detecting light reflection signal intensity. When antibody-drug conjugates effectively inhibit cell growth or induce apoptosis, mechanical force of multicellular spheroids will significantly decrease.

**Example 50: Determining Antibody-Drug Conjugate Target Specificity by Simultaneously Comparing Mechanical Force Changes Between Tumor and Non-Tumor Cells**

This example aims to provide a method for determining antibody-drug conjugate target specificity by monitoring cellular mechanical force.

S1. Prepare cellular mechanical force detection device with microcolumn tops coated with extracellular matrix; treat microcolumn sides and spaces between microcolumns with Pluronic F127 for anti-adhesion.

S2. Mix red-fluorescent-labeled tumor cells and green-fluorescent-labeled non-tumor cells in equal proportions, culture on the detection device for one day to ensure complete attachment. Add antibody-drug conjugates to the culture medium.

S3. Monitor cellular mechanical force of both cell types simultaneously by detecting light reflection signal intensity. When antibody-drug conjugates specifically inhibit tumor cell growth, the decrease in mechanical force will be greater in tumor cells than in non-tumor cells, or non-tumor cells will show no mechanical force changes.

**Example 51: In Vitro Organ Chip**

This example provides an in vitro organ chip, which includes the cellular mechanical force detection device or detection system described in any of the above examples, and further includes: an electrode device. The microcolumns are made of conductive material, and the electrode device acts on the microcolumns to realize electrical stimulation of organs or cells. Organ-related cells and tissues can be seeded on the microcolumns; cellular mechanical force of cells and tissues can deform the microcolumns, converting it into optical signals. The microcolumns are configured with corresponding stiffness and length according to the organ, so that the microenvironment of the chip is suitable for the real structural environment of the organ. The stiffness of microcolumns is controlled by adjusting their length and crosslinking ratio to simulate the stiffness of corresponding tissues or pathological microenvironments (such as cardiac fibrosis).

In some specific examples, to more realistically simulate the microenvironment:

The microcolumn surface is provided with ECM;

A mixture of organ-related cells/tissues and ECM is seeded on the microcolumns;

The microcolumn surface has a tropic ECM coating (e.g., to simulate cardiac ECM microstructure and guide cardiomyocyte orientation);

Seeding methods include 3D printing;

For cardiac applications, cells include one or more combinations of cardiomyocytes, smooth muscle cells, vascular endothelial cells, fibroblasts, stem cells, and immune cells. Microcolumn tops can be coated with decellularized cardiac ECM, artificially recombinant cardiac ECM, or cells can be encapsulated in gels containing cardiac ECM to simulate cardiac extracellular matrix components.

In some specific examples, to simulate mechanical microenvironment:

Includes a mechanical simulation device: a mechanical stretching device for stretching the chip body; or an inflatable deformable flexible membrane at the bottom of microcolumns (upper end connected to microcolumns, lower end connected to base, or base itself is a flexible membrane).

In some specific examples, includes a microfluidic device to apply flow field stimulation to organ-related cells/tissues. Stimuli such as drugs, mechanical force, biochemical factors, electric fields, and flow fields (or combinations) can be applied to identify each cell and organ state under external stimulation.

**Example 52: Method for Establishing In Vitro Organ/Cell Models and Characterizing Cells/Organ Tissues**

S1. Collect microenvironmental parameters of corresponding organ tissues under physiological and pathological states, and prepare the corresponding in vitro organ chip based on these parameters;

S2. Seed cultures containing organ-related cells and/or tissues on the in vitro organ chip;

S3. Optionally add stimuli including drugs, mechanical force, biochemical factors, electric fields, flow fields, or combinations thereof, acting on the culture;

S4. Obtain changes in cellular mechanical force of each cell and tissue through the in vitro organ chip to characterize each cell and organ tissue.

In this example, specific cells or tissues can be sorted through characterization. Uses include screening drugs for organ treatment and studying organ models under physiological and pathological states.

**Example 53: Method for Detecting Cardiomyocyte Contraction-Relaxation Using In Vitro Organ Chip**

This example uses the cellular mechanical force detection device/system, detection method, or in vitro organ chip described in any previous example to obtain cellular physical information:

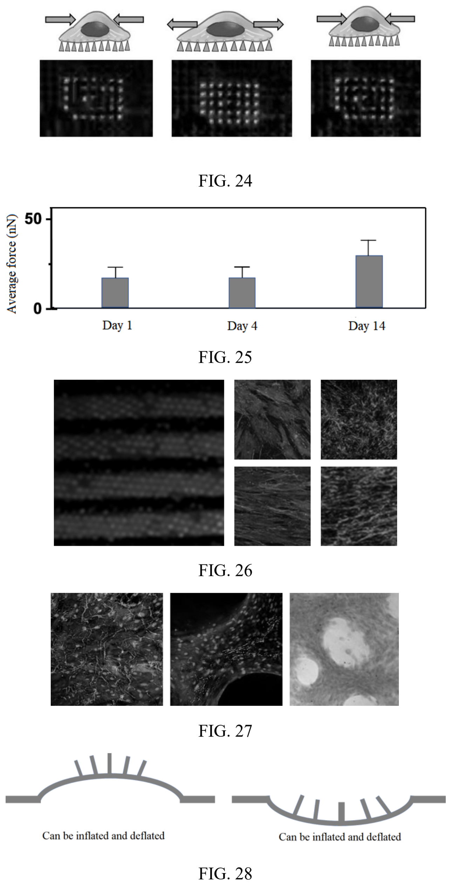

S1. Perfuse mouse heart with high-concentration EDTA solution, mince, and digest into single cells using collagenase;
S2. Allow cell suspension to stand for 20 minutes; cardiomyocytes will sediment to the bottom of the tube;
S3. Directly culture the obtained cardiomyocytes on the in vitro organ chip;
S4. After several days of culture, cardiomyocytes will exhibit regular contractions;
S5. Use high-speed photography to capture 100 images per second, observe mechanical changes during each contraction-relaxation cycle, and calculate contraction frequency after continuously recording multiple cycles (see FIG. 24).

**Example 54: Method for Detecting Drug Effects on Cardiomyocyte Contraction-Relaxation Frequency Using In Vitro Organ Chip**

This example uses the cellular mechanical force detection device/system, detection method, or in vitro organ chip described in any previous example to obtain cellular physical information:

S1. Perfuse mouse heart with high-concentration EDTA solution, mince, and digest into single cells using collagenase;
S2. Allow cell suspension to stand for 20 minutes; cardiomyocytes will sediment to the bottom of the tube;
S3. Directly culture the obtained cardiomyocytes on the in vitro organ chip;
S4. After several days of culture, cardiomyocytes will exhibit regular contractions;
S5. Use high-speed photography to capture 100 images per second, observe mechanical changes during each contraction-relaxation cycle, and calculate contraction frequency after continuously recording multiple cycles;
S6. Add calcium channel blocker Nifedipine, continuously record cardiomyocyte mechanical changes, and observe slowed contraction frequency.

**Example 55: Method for Printing Extracellular Matrix Patterns on In Vitro Organ Chip to Study Cardiomyocyte Alignment and Contraction Frequency**

This example uses the cellular mechanical force detection device/system, detection method, or in vitro organ chip described in any previous example to obtain cellular physical information:

S1. Use microcontact printing to print extracellular matrix patterns on the mechanical chip;
S2. Culture rat cardiomyocytes on the chip; cells will adhere and grow in ECM-patterned regions, forming ordered aligned structures;
S3. After several days of culture, cardiomyocytes will exhibit regular contractions;
S4. Use high-speed photography to capture 100 images per second, observe mechanical changes during each contraction-relaxation cycle, and calculate contraction frequency after continuously recording multiple cycles.

**Example 56: Method for Measuring Mechanical Force Changes During Fibroblast-to-Adipocyte Differentiation Using In Vitro Organ Chip**

This example uses the cellular mechanical force detection device/system, detection method, or in vitro organ chip described in any previous example to obtain cellular physical information:

S1. Culture mouse fibroblast cell line NIH3T3-L1 on the mechanical chip and start recording cellular mechanical force;
S2. Replace culture medium with medium containing methylisobutylxanthine, dexamethasone, and insulin;
S3. On day 3, replace medium with insulin-containing medium;
S4. On day 6, replace medium with normal DMEM medium;
S5. Around day 10, NIH3T3-L1 cells will differentiate into adipocytes. Oil Red O staining monitors lipid accumulation to identify differentiated adipocytes, and Calcein AM staining excludes dead cells;
S6. Record cellular mechanical force changes throughout the differentiation process (see FIG. 25).

**Example 57: Comparing Cellular Mechanical Forces of Brown and White Adipocytes**

This example uses the cellular mechanical force detection device/system, detection method, or in vitro organ chip described in any previous example to obtain cellular physical information:

S1. Isolate brown adipose tissue and white adipose tissue from newborn mice, mince, digest into single cells with collagenase, filter, and centrifuge to obtain preadipocytes;

S2. Culture and expand preadipocytes from brown and white adipose tissues separately in culture dishes;

S3. After several days of culture, transfer both types of preadipocytes to mechanical chips for culture and start recording cellular mechanical force;

S4. Add medium containing methylisobutylxanthine, dexamethasone, and insulin to preadipocytes isolated from white adipose tissue to differentiate into white adipocytes. Add medium containing methylisobutylxanthine, dexamethasone, insulin, and triiodothyronine to preadipocytes isolated from brown adipose tissue to differentiate into brown adipocytes;

S5. After two days of culture, replace medium with insulin-containing medium; for brown adipocyte differentiation, add triiodothyronine;

S6. Use Oil Red O staining to monitor lipid accumulation and identify differentiated adipocytes; use Calcein AM staining to exclude dead cells;

S7. Record cellular mechanical force changes throughout differentiation and compare mechanical forces between brown and white adipocytes.

**Example 58: Stem Cell-Induced Cardiac Organ Model and Characterization Method**

This example provides a stem cell-induced cardiac model by printing tropic ECM on a cellular mechanical force detection device using microcontact printing, seeding stem cells, and inducing cardiomyocyte differentiation. During induction, monitor changes in cellular mechanical force and stiffness using light reflection signals and magnetic field-induced microcolumn deflection.

S1. Prepare tropic extracellular matrix (ECM) on mechanical chip microcolumn tops using microcontact printing technology;

S2. Perform anti-adhesion treatment on microcolumn sides and between microcolumns (e.g., using Pluronic F127);

S3. Seed stem cells on the mechanical chip with tropic ECM and allow complete attachment;

S4. Add appropriate induction factors to guide stem cell differentiation into cardiomyocytes;

S5. During induction, monitor cell-microcolumn interactions using light reflection signals to detect real-time changes in cellular mechanical force;

S6. Use magnetic field-induced microcolumn deflection to measure forces exerted by cells and further evaluate changes in cell stiffness;

S7. Analyze light reflection signals and magnetic field-induced microcolumn deflection data to evaluate changes in cellular mechanical force and stiffness during cardiomyocyte differentiation;

S8. Perform fluorescent staining on cells, extracellular matrix, and corresponding controls to confirm cell and ECM orientation characteristics (FIG. 26).

This stem cell-induced cardiac chip provides a powerful tool for cardiomyocyte differentiation research.

**Example 59: Stem Cell-Induced Tissue-Specific Cartilage Model and Characterization Method**

This example provides a stem cell-induced cartilage model by coating cartilage-specific ECM extracted from cartilage tissue on a chip, seeding adult stem cells (MSCs), and inducing chondrogenic differentiation. During induction, monitor changes in cellular mechanical force and stiffness using light reflection signals and magnetic field-induced microcolumn deflection, and compare with fluorescent and histochemical staining results.

S1. Coat microcolumn tops of mechanical chip with cartilage-specific extracellular matrix (ECM) extracted from cartilage tissue;

S2. Perform anti-adhesion treatment on microcolumn sides and between microcolumns (e.g., using Pluronic F127);

S3. Seed adult stem cells (MSCs) on the mechanical chip coated with cartilage-specific ECM and allow complete attachment;

S4. Add appropriate induction factors to guide adult stem cell differentiation into chondrocytes;

S5. During induction, monitor cell-microcolumn interactions using light reflection signals to detect real-time changes in cellular mechanical force;

S6. Use magnetic field-induced microcolumn deflection to measure forces exerted by cells and further evaluate changes in cell stiffness;

S7. Perform fluorescent staining and histochemical staining during differentiation to evaluate expression of chondrogenic differentiation markers;

S8. Analyze light reflection signals and magnetic field-induced microcolumn deflection data, compare with fluorescent and histochemical staining results to evaluate changes in cellular mechanical force and stiffness during chondrogenic differentiation, providing a powerful tool for chondrocyte differentiation research (FIG. 27).

**Example 60: Lung Tumor Model and Characterization Method**

This example provides a lung tumor model and characterization method by seeding normal and tumor lung cells on a double-sided sandwich-structured chip with a flexible base that inflates/deflates to simulate pulmonary respiration. During chemotherapy drug stimulation, monitor changes in cellular mechanical force and stiffness using light reflection signals and magnetic field-induced microcolumn deflection to measure tumor killing efficacy and effects on normal cells for evaluating drug targeting.

S1. Prepare double-sided sandwich-structured mechanical chip with flexible base; coat microcolumn tops with extracellular matrix (ECM); perform anti-adhesion treatment on microcolumn sides, between microcolumns, and on base (e.g., using Pluronic F127);

S2. Seed normal lung cells on one side of the chip and lung tumor cells on the other side (or mixed in different regions on the same side) and allow complete attachment;

S3. Place the double-sided chip in an inflatable/deflatable device to simulate pulmonary respiration (FIG. 28);

S4. Add chemotherapy drugs to stimulate normal lung cells and lung tumor cells;

S5. During stimulation, monitor cell-microcolumn interactions using light reflection signals to detect real-time changes in cellular mechanical force;

S6. Use magnetic field-induced microcolumn deflection to measure forces exerted by cells and further evaluate changes in cell stiffness;

S7. Analyze light reflection signals and magnetic field-induced microcolumn deflection data, compare effects of chemotherapy drugs on normal lung cells and lung tumor cells, measure tumor killing efficacy and effects on normal cells;

S8. Evaluate targeting of chemotherapy drugs based on experimental results, providing basis for drug screening and research.

**Example 61: Method for Distinguishing Tumor and Non-Tumor Regions in Tissue**

This example uses the cellular mechanical force detection device/characterization system or detection method described in any previous example to obtain cellular physical information from different tissues, including the following steps:

S1. Inject pancreatic cancer cell line into mouse pancreas; after several weeks to allow tumor formation, remove tumor-containing tissues (e.g., pancreas and liver) from mice; cut tissues into ~0.5 cm $\times$ 0.5 cm pieces; fix tissues on tissue embedding plate using adhesive; section into 150-200 $\mu$m thick slices using a microtome;

S2. Place tissue slices on microcolumns of cellular detection device; add cell culture medium to just cover tissue slices; after 30 minutes, add more medium to completely submerge tissues; start monitoring tissue cellular mechanical force and changes after 1 hour;

S3. Detect cellular mechanical force to identify regions with strong and weak mechanical force in the tissue.

In control validation:

Pancreatic cancer cell line in step S1 expresses EGFP fluorescent protein;

Compare EGFP signals with mechanical force intensity in step S3, revealing that cancer cell-containing regions (EGFP-expressing regions) exhibit stronger cellular mechanical force.

Thus, validation confirms that regions with strong cellular mechanical force contain cancer cells, enabling identification of different tissue regions (FIG. 31).

**Example 62: Monitoring Tissue Response to Drugs Through Cellular Mechanical Force**

This example uses the cellular mechanical force detection device/characterization system or detection method described in any previous example to obtain cellular physical information from different tissues, including the following steps:

S1. Inject pancreatic cancer cell line expressing EGFP fluorescent protein into mouse pancreas;
S2. After several weeks to allow tumor formation, remove tumor-containing tissues (e.g., pancreas and liver) from mice;
S3. Cut tissues into ~0.5 cm $\times$ 0.5 cm pieces;
S4. Fix tissues on tissue embedding plate using adhesive; section into ~150-200 micrometer thick slices using a microtome;
S5. Place tissue slices on mechanical chip; add cell culture medium to just cover tissue slices;
S6. After 30 minutes, add more medium to completely submerge tissues; start recording and monitoring tissue mechanical changes after 1 hour;
S7. After ~6 hours, when tissues are completely attached to the chip, add drugs for testing (e.g., gemcitabine and 5-FU, commonly used for pancreatic cancer);
S8. Continuously monitor cellular mechanical force changes across the entire tissue. Since cellular mechanical force decrease significantly or disappear when cells undergo apoptosis or death, observe whether and to what extent tumor region mechanical force decreases to predict drug therapeutic efficacy.

**Example 63: Spatial Omics Monitoring of Mechanical Properties in Living Tissue Sections for Distinguishing Tumor/Normal Tissues and Evaluating Drug Treatment Efficacy**

This example provides a tissue biophysical characterization system to distinguish tumor and normal tissue regions and evaluate drug treatment efficacy by monitoring spatial omics of mechanical properties in living tissue sections.

S1. Prepare living tissue sections: obtain living tissue samples (containing tumor and normal tissues) and prepare thin sections;
S2. Measure mechanical properties of tissue sections: monitor mechanical force through light reflection signals from mechanical chip, measure stiffness through magnetic field-activated deflection of microcolumns inserted into tissue, and validate with atomic force microscopy (AFM) or similar techniques;
S3. Distinguish tumor and normal tissues: analyze spatial omics data of tissue section mechanical properties to distinguish tumor and normal tissue regions (FIG. 29). Tumor regions exhibit greater mechanical force and higher stiffness;
S4. Drug treatment: add anti-tumor drugs to living tissue sections and incubate for a certain period. Immune cell suspensions can also be added to measure tumor-immune interactions;
S5. Evaluate drug treatment efficacy: after drug treatment, measure changes in mechanical force and stiffness at tumor locations. Observe significant decrease in mechanical force at tumor locations (FIG. 30) with no significant change in stiffness, indicating that mechanical force responds earlier than stiffness.

Finally, it should be noted that the above examples are only for illustrating the technical solutions of the present invention and not for limiting them; although the present invention has been described in detail with reference to the foregoing examples, those of ordinary skill in the art should understand that: it is still possible to modify the technical solutions described in the foregoing examples or replace some or all technical features equivalently; and these modifications or replacements do not make the essence of corresponding technical solutions depart from the scope of the technical solutions of the examples of the present invention.

**Claims**

1. A method for characterizing cells, comprising characterizing cells and/or multicellular aggregates by acquiring cellular physical information of the cells and/or the multicellular aggregates.

2. The method for characterizing cells according to claim 1, wherein the cellular physical information comprises cellular mechanical force and/or hardness obtained under at least one of the following conditions:

(1) an interaction between the cells and the multicellular aggregates;

(2) an interaction between the cells;

(3) an interaction between the multicellular aggregates;

(4) cells and/or multicellular aggregates at different growth times;

(5) different regions within the multicellular aggregates;

(6) an effect of a substance on the cells and/or the multicellular aggregates; and

(7) an effect of other physical, biological or chemical factors on the cells and/or the multicellular aggregates;

wherein the multicellular aggregate is a cell population formed by aggregation of more than two cells.

3. The method for characterizing cells according to claim 2, wherein

the cellular mechanical force comprises one or a combination of two or more of magnitude, direction, frequency, and distribution;

optionally, the hardness comprises hardness and/or distribution of the cells and/or the multicellular aggregates; and

optionally, the cellular mechanical force and/or the hardness comprises cellular mechanical force and/or hardness at a certain point.

4. The method for characterizing cells according to claim 2, wherein the cellular physical information comprises a change in the cellular mechanical force and/or the hardness within a certain time interval.

5. The method for characterizing cells according to claim 2, wherein the cellular physical information further comprises cell morphology information.

6. The method for characterizing cells according to claim 2, wherein the cellular physical information is obtained under a cell confinement operation on the cells and/or the multicellular aggregates.

7. The method for characterizing cells according to claim 2, wherein

the interaction between the cells and the multicellular aggregates comprises: an interaction between the cells and extracellular matrix, a direct interaction between the cells through an intercellular connecting substance, and an interaction through an intercellular signal molecule;

the interaction between the cells comprises: intercellular signal transduction and intercellular mutual recognition;

the interaction between the multicellular aggregates comprises: an interaction between different tissues and an interaction between different organs;

the cells and/or the multicellular aggregates at different growth times comprise: an interaction between embryonic cells at different developmental stages, and tissue regeneration and repair;

the different regions within the multicellular aggregates comprise: formation and maintenance of cell polarity in a multicellular tissue, comprising regulation of distribution of a polarity protein within cells, and a site of a cell-cell interaction;

the effect of the substance on the cells and/or the multicellular aggregates comprises: an interaction between a drug and the cells, and an interaction between a toxin and the cells; and

the effect of other physical, biological or chemical factors on the cells and/or the multicellular aggregates comprises: an influence of temperature and pressure on the cells, and an influence of light on plant cells.

8. The method for characterizing cells according to claim 2, wherein the method is based on the cellular physical information obtained under the interaction between the cells and the multicellular aggregates, the interaction between the cells, or the interaction between the multicellular aggregates, comprises the steps of:

placing first cells and/or multicellular aggregates and second cells and/or multicellular aggregates in a specific region, detecting and acquiring cellular physical information;

or

after placing first cells and/or multicellular aggregates in a specific region, interacting second cells and/or multicellular aggregates with the first cells and/or multicellular aggregates, detecting and acquiring cellular physical information;

wherein the first cells and/or multicellular aggregates and the second cells and/or multicellular aggregates are all one or more cells and/or multicellular aggregates.

9. The method for characterizing cells according to claim 2, wherein the substance comprises one or a combination of two or more of a biologically active macromolecule, a chemical substance, a biologically active substance, and an inactivated biological substance; and the biologically active macromolecule comprises one or a combination of two or more of a protein, a polypeptide, a polysaccharide, and a fat.

10. The method for characterizing cells according to claim 2, wherein the method is based on the cellular physical information during implantation and growth of an in vitro organ and/or a related cell model; and the cellular physical information comprises:

   cellular physical information of cells and/or any region during implantation and culture of organ-related cells and/or tissue cultures;
   optionally, cells or cell clusters, organ tissues in any region during growth of an in vitro organ and/or a related cell model are characterized through the cellular physical information;
   optionally, a mixture of cells and/or tissues is implanted with ECM;
   optionally, a method for the implantation comprises: performing a 3D printing and/or microfluidic method to control distribution and flow of cells and biological materials to achieve implantation;
   optionally, the organ-related cells and/or the tissue cultures are implanted on a characterization device; and optionally, the detection device is provided with an ECM tropism coating.

11. The method for characterizing cells according to claim 10, wherein the detection device is customized according to microenvironmental parameters of corresponding tissues in physiological and pathological states.

12. The method for characterizing cells according to any one of claims 1-11, comprising the steps of:

   after placing the cells and/or the multicellular aggregates in a specific region, and applying at least one combination of physical stimulation, biological stimulation, and chemical stimulation to the cells and/or the multicellular aggregates; and
   detecting and acquiring the cellular physical information of the cells and/or the multicellular aggregates;
   wherein optionally, the physical stimulation, the biological stimulation, and the chemical stimulation comprise: one or a combination of two or more of a drug, mechanical force, hardness, biochemistry, electric field, flow field, tropism guidance, and radiation.

13. The method for characterizing cells according to any one of claims 1-11, wherein the cellular physical information is acquired before, during and/or after the action of the stimulation; or the cellular physical information is acquired at different growth times of the cells and/or the multicellular aggregates.

14. The method for characterizing cells according to any one of claims 1-11, wherein the cellular physical information is acquired through real-time monitoring.

15. The method for characterizing cells according to any one of claims 1-11, wherein the cellular physical information is acquired through a characterization system;
   the characterization system comprises a characterization device, comprising:

   a base, and
   a microcolumn array composed of one or more microcolumns, arranged on the base and capable of undergoing deformation under the action of cellular mechanical force and/or magnetic force, and the microcolumn provided with a light reflection layer;
   wherein optionally, an end of the microcolumn away from the base is provided with a light reflection layer, the base is provided with a light-transmitting part, and a column body of the microcolumn is provided with a light-transmitting part; and optionally, a surface of the microcolumn and/or the base has an anti-reflection layer.

16. The method for characterizing cells according to claim 15, wherein

   the characterization system further comprises a light signal emitting device and a light signal detecting device, light emitted by the light signal emitting device irradiates the light reflection layer through an incident light path, and light reflected by the light reflection layer enters the light signal detecting device through a reflected light path;
   optionally, the optical intensity acquired by the light signal detecting device is analyzed to obtain cellular physical information;

optionally, the optical intensity acquired by the light signal detecting device has a linear correlation with magnitude of the cellular mechanical force, enabling qualitative and quantitative analysis to realize different cell typing.

17. The method for characterizing cells according to claim 15, wherein the characterization device is capable of containing a liquid;

if the liquid is a cell culture medium, the cells and/or the multicellular aggregates are attached and/or continuously cultured; and
optionally, the cells and/or the multicellular aggregates are adhered to the microcolumns through an adhesive substance provided on the microcolumns.

18. A method for cell typing and identification, comprising performing typing and identification of the cells and/or the multicellular aggregates through the cellular physical information according to any one of claims 1-11;

wherein optionally, the typing and identification comprise: a type, a state, a behavior, a spatial omics characteristic and a differentiation direction, and a stress response of the cells and/or the multicellular aggregates; and
optionally, the typing and identification comprise: selectively sorting out specific cells or tissues through the cellular physical information.

19. The method for cell typing and identification according to claim 18, comprising using a cell identification device for identification, wherein the cell identification device comprises an information acquisition unit, a preprocessing unit, a learning unit, and an identification unit;

the information acquisition unit is configured to acquire the cellular physical information of the cells and/or the multicellular aggregates;
the preprocessing unit is configured to preprocess the cellular physical information to form structured cellular physical information; and the structured cellular physical information comprises the number of cells, the number of cell features, and feature information of each cell feature;
the learning unit is configured to establish a cell feature model using supervised, unsupervised or semi-supervised machine learning with the structured cellular physical information as input data; and
the identification unit is configured to apply the cell feature model to classification or clustering of the cells and/or the multicellular aggregates to realize the typing and identification of the cells and/or the multicellular aggregates.

20. The method for cell typing and identification according to claim 19, wherein

the typing and identification comprise at least one combination of the following: typing and identification of tumor regions and non-tumor regions in tissues;
typing and identification of cells and/or multicellular aggregates with different transfection degrees and non-transfected ones; and
typing and identification of cells and/or multicellular aggregates with different yields of biologically active substances; and
monitoring an influence of genetic engineering on cells comprises: identifying small clone clusters or single cells from large clone clusters;
determining a minimum effective concentration of a drug to kill non-resistant cells; performing real-time monitoring of the cells during drug screening for timely adjustment; and
monitoring growth and differentiation of adipocytes, optionally for real-time monitoring of a conversion process of white fat, beige fat, and brown fat.

21. Application of the method according to any one of claims 1-11, 18-20, comprising at least one of the following:

application in establishing an in vitro organ and a cell model;
application in screening an organ therapeutic drug, and in research on an organ model in physiological and pathological states;
application in a method for evaluating drug efficacy against a tumor and a related evaluation product; and
use in cell therapy, synthetic biology, adipose research, research on an interaction between cells/multicellular aggregates and macromolecules, a research method for multicellular aggregates, and a related product thereof.

FIG. 1

FIG. 2

FIG. 3

FIG. 4

FIG. 5

FIG. 6

FIG. 7

FIG. 8

FIG. 9

Microwell A459 cell membrane (Dil) Light reflection

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

Can be inflated and deflated

Can be inflated and deflated

FIG. 28

FIG. 29

FIG. 30

FIG. 31

## INTERNATIONAL SEARCH REPORT

| International application No. |
|---|
| **PCT/CN2024/083362** |

**A. CLASSIFICATION OF SUBJECT MATTER**

C12M 1/34(2006.01)i; C12M 1/00(2006.01)i; C12Q 1/02(2006.01)i; G06V20/69(2022.01)i

According to International Patent Classification (IPC) or to both national classification and IPC

**B. FIELDS SEARCHED**

Minimum documentation searched (classification system followed by classification symbols)

IPC:C12M,C12Q,G06V

Documentation searched other than minimum documentation to the extent that such documents are included in the fields searched

Electronic data base consulted during the international search (name of data base and, where practicable, search terms used)

CJFD, CNTXT, ENTXT, ENTXTC, PUBMED, SpringerLink, ISI Web of Knowledge, Elsevier Science Direct, Wiley InterScience, Nature, Science, CNKI, 万方, WANFANG: 细胞机械力, 细胞牵引力, 细胞力, 微柱, 反射, 物理信息, 识别, mechanical force, micro-column, array, cell recognition, traction force, mechanical.

**C. DOCUMENTS CONSIDERED TO BE RELEVANT**

| Category* | Citation of document, with indication, where appropriate, of the relevant passages | Relevant to claim No. |
|---|---|---|
| PX | WO 2023046167 A1 (RUIXIN (FUZHOU) TECHNOLOGY CO., LTD) 30 March 2023 (2023-03-30) claims 1-15 | 1-21 |
| X | 熊春阳 (XIONG, Chunyang). "细胞力学信息学-从技术到应用 (non-official translation: Cell Mechanics Informatics-From Technology to Application)" 第十三届全国生物力学学术会议论文摘要汇编 *(non-official translation: The 13th National Conference on Biomechanics Abstracts)*, Vol. 36, No. S1, 31 December 2021 (2021-12-31), page 71 | 1 |
| Y | US 2019317050 A1 (HUNAN AGRICULTURAL UNIVERSITY) 17 October 2019 (2019-10-17) description, paragraphs 4 and 21-32 | 2-21 |
| Y | US 2015300953 A1 (THE UNIVERSITY OF NORTH CAROLINA AT CHAPEL HILL) 22 October 2015 (2015-10-22) claims 1-83, description, paragraphs 16-63, and figure 1 | 15-17 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 June 2024** | **03 July 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/083362** |

**C.    DOCUMENTS CONSIDERED TO BE RELEVANT**

| Category* | Citation of document, with indication, where appropriate, of the relevant passages | Relevant to claim No. |
|---|---|---|
| Y | US 2018372635 A1 (THE REGENTS OF THE UNIVERSITY OF CALIFORNIA) 27 December 2018 (2018-12-27)<br>      claims 1-51 | 15-17 |
| Y | 熊春阳 (XIONG, Chunyang). "细胞力学信息学-从技术到应用 (non-official translation: Cell Mechanics Informatics-From Technology to Application)"<br>*第十三届全国生物力学学术会议论文摘要汇编 (non-official translation: The 13th National Conference on Biomechanics Abstracts)*, Vol. 36, No. S1, 31 December 2021 (2021-12-31),<br>      page 71 | 2-21 |
| Y | 李宁 等 (LI, Ning et al.). "细胞力学2021年度研究进展 (Progress of Cell Biomechanics in 2021)"<br>*医用生物力学 (Journal of Medical Biomechanics)*,<br>Vol. 37, No. 4, 31 August 2022 (2022-08-31),<br>      pages 585 and 589 | 2-21 |
| Y | 李泽诚 (LI, Zecheng). "细胞力学特性测量研究及测量平台研制 (Study on Cell Mechanical Properties with A Homemade Platform)"<br>*中国优秀硕士学位论文全文库医药卫生科技 (Medicine and Health Sciences, China Master's Theses Full-Text Database)*, 15 January 2021 (2021-01-15),<br>      abstract, sections 2.1-2.2, 3.1, and 4.1 | 2-21 |

Form PCT/ISA/210 (second sheet) (July 2022)

**INTERNATIONAL SEARCH REPORT**
Information on patent family members

International application No.

**PCT/CN2024/083362**

| Patent document cited in search report | | | Publication date (day/month/year) | Patent family member(s) | | | Publication date (day/month/year) |
|---|---|---|---|---|---|---|---|
| WO | 2023046167 | A1 | 30 March 2023 | AU | 2022350581 | A1 | 09 May 2024 |
| US | 2019317050 | A1 | 17 October 2019 | JP | 2019530442 | A | 24 October 2019 |
| | | | | JP | 6851651 | B2 | 31 March 2021 |
| | | | | EP | 3505925 | A1 | 03 July 2019 |
| | | | | EP | 3505925 | A4 | 08 April 2020 |
| | | | | EP | 3505925 | B1 | 28 June 2023 |
| | | | | WO | 2018041060 | A1 | 08 March 2018 |
| | | | | US | 11029285 | B2 | 08 June 2021 |
| | | | | AU | 2017319889 | A1 | 18 April 2019 |
| | | | | AU | 2017319889 | B2 | 06 January 2022 |
| US | 2015300953 | A1 | 22 October 2015 | US | 9952149 | B2 | 24 April 2018 |
| | | | | WO | 2014085804 | A1 | 05 June 2014 |
| | | | | EP | 2926115 | A1 | 07 October 2015 |
| | | | | EP | 2926115 | A4 | 06 July 2016 |
| | | | | EP | 2926115 | B1 | 26 May 2021 |
| US | 2018372635 | A1 | 27 December 2018 | WO | 2017015063 | A1 | 26 January 2017 |
| | | | | US | 10712271 | B2 | 14 July 2020 |

Form PCT/ISA/210 (patent family annex) (July 2022)